# EUROPEAN PATENT APPLICATION

(11) **EP 3 826 430 A1**
(43) Date of publication of application: **26.05.2021**
(21) Application number: 19837198.1
(22) Date of filing: 17.07.2019
(51) Int. Cl.: H05B 33/02, H01L 51/50

(54) **ORGANIC ELECTROLUMINESCENT ELEMENT**

(30) Priority: 17.07.2018 JP 2018134445
(71) Applicant: Hodogaya Chemical Co., Ltd., Tokyo 104-0028 (JP)
(72) Inventor: KASE Kouki, Tokyo 104-0028 (JP); MOCHIZUKI Shunji, Tokyo 104-0028 (JP); HIRAYAMA Yuta, Tokyo 104-0028 (JP)
(74) Representative: Müller-Boré & Partner Patentanwälte PartG mbB
(86) International application number: PCT/JP2019/028088
(87) International publication number: WO 2020/017552

(57) **Abstract**

The present invention is an organic EL element in which a material used for a capping layer is a material having a high absorbance as measured at a wavelength of 400 to 410 nm in an absorption spectrum at a concentration of 10⁻⁵ mol/L, and the material is selected from an arylamine material which provides excellent stability and durability to a thin film, particularly selected from a diamine compound that has a particular benzazol ring structure having a high refractive index.

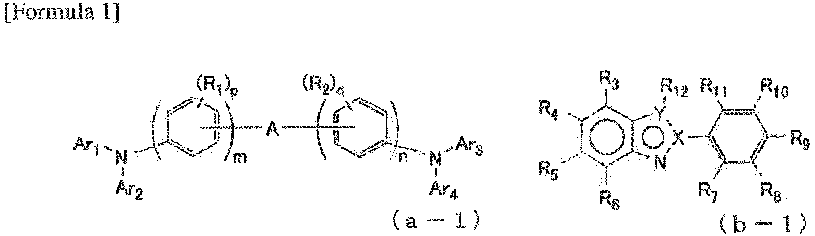

## Description

### TECHNICAL FIELD

The present invention relates to an organic electroluminescent element (which hereinafter may be abbreviated as an organic EL element) and a method for manufacturing the same.

This application claims priority based on Japanese Patent Application No. 2018-134445 filed July 17, 2018, the contents of which are incorporated herein by reference.

### BACKGROUND TECHNOLOGY

The organic EL element is a self-luminous element. In addition, the organic EL element is brighter and has better visibility than a liquid crystal element, and a clear display is possible. Therefore, organic EL elements have been actively studied.

In 1987, C. W. Tang et al. of Eastman Kodak Co. developed a multilayer structure element which shared various roles to each material. As a result, they made an organic EL element using an organic material practical. They laminate a phosphor capable of transporting electrons and an organic material capable of transporting holes, and injected both charges into the phosphor layer to emit light. As a result, the organic EL element can obtain a high luminance of 1000cd/m² or more at a voltage of 10V or less (see Patent Document 1 and Patent Document 2).

Until now, many improvements have been made to the practical application of organic EL elements, and the various roles of the laminated structure have been further subdivided. High efficiency and durability are achieved by an electroluminescent element in which an anode, a hole injection layer, a hole transport layer, a light emitting layer, an electron transport layer, an electron injection layer, and a cathode are sequentially provided on the substrate and which has a bottom emission structure emitting light from the bottom thereof (for example, see Non-Patent Document 1).

In recent years, a light emitting element having a top emission structure which emits light from an upper portion by using a metal having a high work function as an anode has been used. In the bottom emission structure for extracting light from the bottom portion having a pixel circuit, the area of the light emitting portion is limited. On the other hand, in the light emitting element of the top emission structure, since light is extracted from the upper portion, light from the light emitting portion is not blocked by the pixel circuit. Therefore, a light emitting element having a top emission structure has an advantage in that a light emitting portion can be made wide. In the light emitting element having the top emission structure, a translucent electrode such as LiF/Al/Ag (for example, see Non-Patent Document 2), Ca/Mg (for example, see Non-Patent Document 3), LiF/MgAg, or the like is used as a cathode.

In such a light emitting element, when light emitted from the light emitting layer enters another film at a certain angle or higher, the light is totally reflected at the interface between the light emitting layer and the other film. Therefore, only a part of the emitted light was usable. In recent years, in order to improve light extraction efficiency, there has been proposed a light emitting element in which a "capping layer" having a high refractive index is provided outside a translucent electrode having a low refractive index (for example, see Non-Patent Documents 2 and 3).

The effect of the capping layer in the light emitting element of the top emission structure was recognized as follows. In a light emitting element using Ir(ppy)₃ as a light emitting material, the light emitting efficiency was 38 cd/A in the absence of a capping layer. On the other hand, in a light emitting element using ZnSe having a thickness of 60 nm as a capping layer, it was observed that the light emitting efficiency was 64 cd/A, which was improved by about 1.7 times. It was also shown that the maximum point of transmittance of the translucent electrode and the capping layer and the maximum point of efficiency do not always coincide with each other. It has been shown that the maximum point of light extraction efficiency is determined by the interference effect (for example, see Non-Patent Document 3).

Conventionally, it has been proposed to use a metal mask having a high definition for forming the capping layer. However, when the metal mask is used under a high temperature condition, there is a problem that the alignment accuracy is lowered because the metal mask is distorted by heat. ZnSe has a high melting point of 1100°C or more (for example, see Non-Patent Document 3). Therefore, even when a metal mask having a high definition is used, the metal mask is distorted when ZnSe is deposited, so that the metal mask cannot be deposited at an accurate position, and there is a possibility that the light emitting element itself is affected. Further, even in the case of forming a ZnSe film by a sputtering method, the effect is exerted on the light emitting element. Therefore, an inorganic substance is not suitable as a constituent material of the capping layer.

In addition, it has been proposed to use tris(8-hydroxyquinoline) aluminum (hereinafter abbreviated as Alq₃) as a capping layer for adjusting the refractive index (for example, see Non-Patent Document 2). Alq₃ is known as an organic EL material commonly used as a green-light-emitting or electron transport material. Alq₃ has a weak absorption near 450 nm, which is used for blue-light emitting materials. Therefore, in the blue-light emitting element using Alq₃ as a capping layer, there is a problem that color purity is lowered and light extraction efficiency is lowered.

Further, in a conventional element having a capping layer, since light having a wavelength of 400 nm to 410 nm of sunlight passes through the element and affects the material inside the element, there is a problem that color purity and light extraction efficiency are lowered.

In order to improve the element characteristics of the organic EL element, it is required that the capping layer absorbs light having a wavelength of 400 nm to 410 nm of sunlight and does not affect the material inside the element. In order to greatly improve the light extraction efficiency of the organic EL element, a material having a high absorption coefficient, a high refractive index, and excellent stability and durability of a thin film is required as a material of a capping layer.

### [Patent Documents]

[Patent Document 1] Japanese Patent Application Laid-Open No. H08-048656
[Patent Document 2] Japanese Patent No. 3194657
[Patent Document 3] WO 2014/009310
[Patent Document 4] WO 2013/038627

### [Non-Patent Documents]

[Non-Patent Document 1] The 9th Session of the Japan Society of Applied Physics, Summary of Previews, pages 55 to 61 (2001)
[Non-Patent Document 2] Appl. Phys. Let., 78,544 (2001)
[Non-Patent Document 3] Appl. Phys. Let., 82,466 (2003)
[Non-Patent Document 4] J. Org. Chem., 71, 1802 (2006)
[Non-Patent Document 5] Aust. J. Chem., 45,371 (1992)
[Non-Patent Document 6] J. Org. Chem., 60, 7508 (1995)
[Non-Patent Document 7] Synth. Commun., 11,513 (1981)
[Non-Patent Document 8] Appl. Phys. Lett., 98, 083302 (2011)

### SUMMARY OF THE INVENTION

It is an object of the present invention to provide an organic EL element having high luminance, good light emission efficiency and power efficiency, and a long life.

### [Means to Solve the Problem]

In order to achieve the above object, the present inventors have conducted intensive research as follows.

That is, in consideration of the excellent stability and durability of the thin film of the arylamine-based material, a material having a high absorbance at wavelengths of 400 nm to 410 nm in the absorption spectrum of 10⁻⁵ mol/L concentration was selected from amine compounds which have a specific benzazole ring structure and have a high refractive index. An organic EL element using this material as a material constituting the capping layer was fabricated, and the characteristics of the element were intensively evaluated. As a result, the present invention has been completed.

In other words, according to the present invention, the following organic EL elements are provided.
(1) An organic electroluminescent element comprising an organic layer comprising a light emitting layer which is provided between a first electrode and a second electrode,
   wherein a capping layer is laminated on a surface of the first electrode opposite to the organic layer;
   the first electrode is transparent or translucent; and
   the capping layer comprises a diamine compound having a benzazole ring structure represented by general formula (a-1), 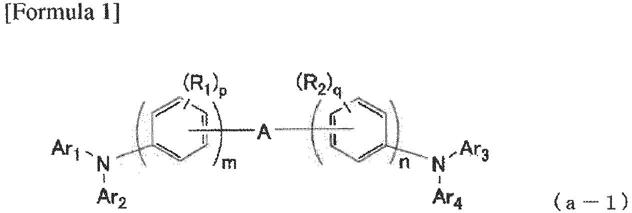 wherein in formula (a-1),
   A represents a divalent group represented by the following general formula (b-1) wherein the divalent group has two binding sites among R₃ to R₁₂;
   Ar₁ to Ar₄ may be the same or different from each other, and each represents a monovalent group represented by general formula (b-1) wherein the monovalent group has one binding site among R₃ to R₁₂, a substituted or unsubstituted aromatic hydrocarbon group, a substituted or unsubstituted aromatic heterocyclic group, or a substituted or unsubstituted condensed polycyclic aromatic group;
   R₁ and R₂ may be the same or different from each other, and may represent hydrogen, deuterium, fluorine, chlorine, cyano, nitro, trimethylsilyl, triphenylsilyl, a linear or branched alkyl having 1 to 6 carbon atoms optionally substituted, a cycloalkyl having 5 to 10 carbon atoms optionally substituted, a linear or branched alkenyl having 2 to 6 carbon atoms optionally substituted, a linear or branched alkyloxy having 1 to 6 carbon atoms optionally substituted, a cycloalkyloxy having 5 to 10 carbon atoms optionally substituted, a substituted or unsubstituted aryloxy, a substituted or unsubstituted aromatic hydrocarbon, a substituted or unsubstituted aromatic heterocyclic, or a substituted or unsubstituted condensed polycyclic aromatic group;
   m and n represent integers of 0 to 2, and p and q represent integers of 0 to 4;
   when m or n is 0, A is bonded to a nitrogen atom as a single bond;
   when m and n are integers of 1 or more, Ar₁, Ar₂, and the benzene ring adjacent to their bonded nitrogen atoms, or Ar₃, Ar₄, and the benzene ring adjacent to their bonded nitrogen atoms may be bonded via the nitrogen atom by a single bond, a substituted or unsubstituted methylene group, a substituted or unsubstituted amino group, an oxygen atom, or a sulfur atom to form a ring;
   when p and q are integers of 2 or more, plurality of R₁ and R₂ bonded to the same benzene ring may be identical or different from each other, and may be bonded to each other through a single bond, a substituted or unsubstituted methylene group, a substituted or unsubstituted amino group, an oxygen atom, or a sulfur atom to form a ring with respect to the same substituted benzene ring, 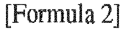 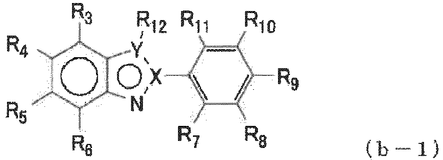 wherein in formula (b-1),
   R₃ to R₁₂ may be the same or different from each other, and each may represent a linking group as a binding site, a hydrogen atom, a deuterium atom, a fluorine atom, a chlorine atom, a cyano group, a nitro group, a trimethylsilyl group, a triphenylsilyl group, a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group;
   X represents a carbon atom or a nitrogen atom;
   Y represents any one of carbon, nitrogen, oxygen, and sulfur atoms;
   provided that, when X is a carbon atom and Y is an oxygen atom, when X is a carbon atom and Y is a sulfur atom, or when X is a nitrogen atom and Y is a nitrogen atom, Y does not have R₁₂.
(2) The organic electroluminescent element according to (1),
   wherein the first electrode is a cathode;
   the second electrode is an anode;
   the organic layer includes at least a hole transport layer, a light emitting layer, and an electron transport layer; and
   the organic EL element comprises the anode, the hole transport layer, the light emitting layer, the electron transport layer, the cathode, and the capping layer, in this order.
(3) The organic electroluminescent element according to claim 1, wherein the general formula (b-1) is the following general formula (b-2), (b-3), or (b-4), 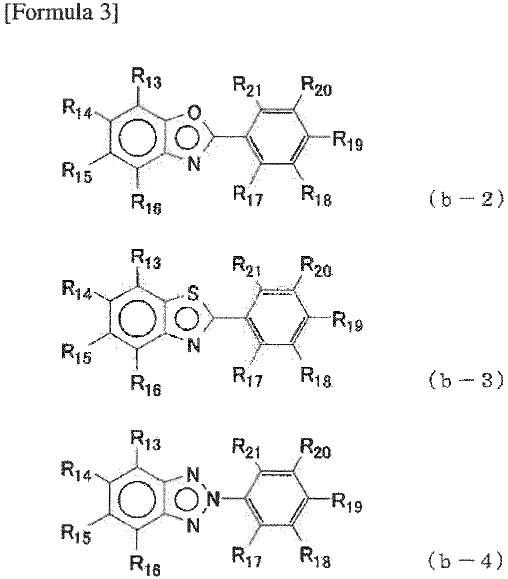 wherein in formulas (b-2), (b-3), or (b-4), R₁₃ to R₂₁ may be the same or different from each other, and each may represent a linking group as a binding site, a hydrogen atom, a deuterium atom, a fluorine atom, a chlorine atom, a cyano group, a nitro group, a trimethylsilyl group, a triphenylsilyl group, a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group.
(4) The organic electroluminescent element according to (1), wherein the general formula (b-1) is the following general formula (b-5), (b-6), or (b-7), 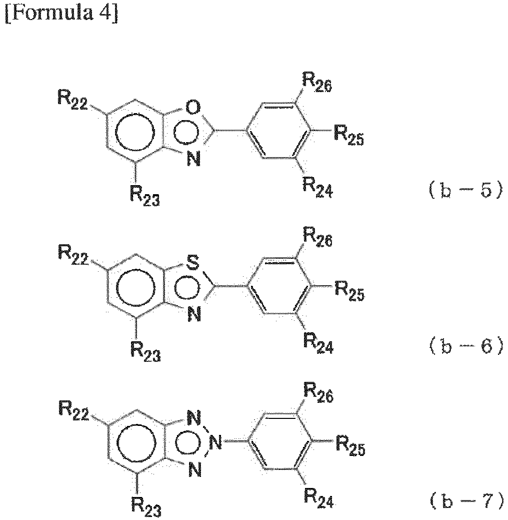 wherein in formulas (b-5),(b-6), or (b-7), R₂₂ to R₂₆ may be the same or different from each other, and each may represent a linking group as a binding site, a hydrogen atom, a deuterium atom, a fluorine atom, a chlorine atom, a cyano group, a nitro group, a trimethylsilyl group, a triphenylsilyl group, a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group.
(5) The organic electroluminescent element according to (1), wherein the general formula (b-1) is the following general formula (b-8), (b-9), or (b-10), 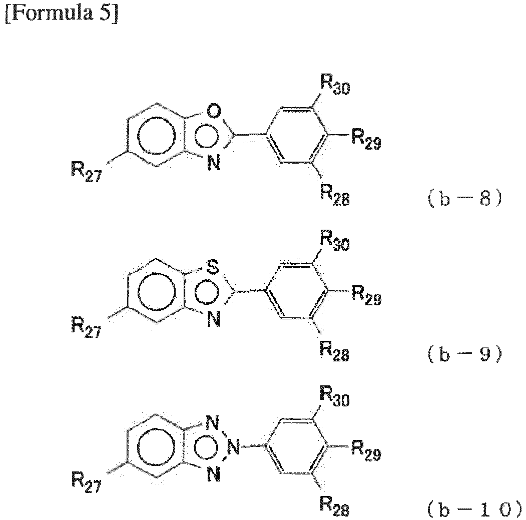 wherein in formulas (b-8), (b-9), or (b-10), R₂₇ to R₃₀ may be the same or different from each other, and each may represent a linking group as a binding site, a hydrogen atom, a deuterium atom, a fluorine atom, a chlorine atom, a cyano group, a nitro group, a trimethylsilyl group, a triphenylsilyl group, a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group.
(6) The organic electroluminescent element according to any one of (1) to (5), wherein the sum of m and n is 0, 1, or 2.
(7) The organic electroluminescent element according to any one of (1) to (6), wherein the two amino group skeletons of -NAr₁Ar₂ and NAr₃Ar₄ in the general formula (a-1) are the same.
(8) The organic electroluminescent element according to any one of (1) to (7), wherein the diamine compound has an absorbance of 0.2 or more in the absorption spectrum of concentration of 10⁻⁵ mol/L in a wavelength range of 400 nm to 410 nm.
(9) The organic electroluminescent element according to any one of (1) to (8), wherein the thickness of the capping layer is in the range of 30 nm to 120 nm.
(10) The organic electroluminescent element according to any one of (1) to (9), wherein the capping layer has a refractive index of 1.85 or more when light having wavelengths of 400 nm and 410 nm is transmitted.
(11) The organic electroluminescent element according to any one of (1) to (10), wherein the capping layer has an extinction coefficient of 0.2 or more for light having wavelengths of 400 nm and 410 nm.
(12) The organic electroluminescent element according to any one of (1) to (11), wherein R1 and R₂ are hydrogen atoms.

### [Effect of the Invention]

The organic EL element of the present invention has high luminance, good light emission efficiency and power efficiency, and a long life.

### BRIEF DESCRIPTION OF THE DRAWING

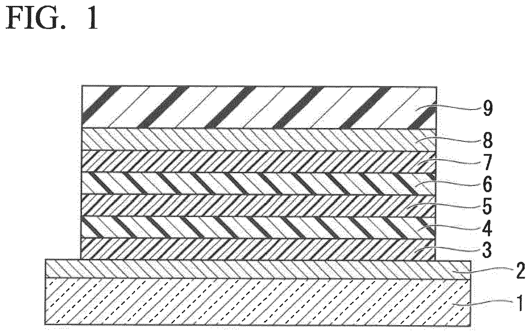

FIG. 1 is a schematic cross-sectional view showing an example of an organic EL element of this embodiment.

### EMBODIMENTS FOR CARRYING OUT THE INVENTION

The purpose of this embodiment is to improve the element characteristics of the organic EL element. In particular, the purpose of this embodiment is an organic EL element which absorbs light having a wavelength of 400 nm to 410 nm and this light does not affect the material inside the element. The object of the present embodiment is to greatly improve the light extraction efficiency of the organic EL element. It is further an object of the present embodiment to provide an organic EL element having a capping layer made of a material (1) having a high absorption coefficient, (2) a high refractive index, (3) good stability of a thin film, (4) excellent durability, (5) excellent light resistance, and (6) no absorption in the respective wavelength regions of blue, green, and red.

Physical characteristics suitable for the capping layer include (1) high absorption coefficient, (2) high refractive index, (3) possible vapor deposition, (4) stable thin film state, and (5) high glass transition temperature.

Physical characteristics suitable for the organic EL element include (1) absorption of light from 400 nm to 410 nm, (2) high light extraction efficiency, (3) no reduction in color purity, (4) transmission of light without change with time, and (5) long lifetime.

### "Organic EL Element"

The organic EL element of this embodiment has an organic layer including a light emitting layer between a first electrode and a second electrode, and a capping layer laminated on the surface of the first electrode opposite to the organic layer.

The organic layer includes a light emitting layer, and may be only the light emitting layer, or may have a laminated structure in which the light emitting layer and the other organic layers are laminated. The organic layer preferably has a light emitting layer disposed between the hole transport layer and the electron transport layer because the light emitting efficiency can be improved by the function-separated structure.

The first electrode is an anode or a cathode. The second electrode is a cathode when the first electrode is an anode, and is an anode when the first electrode is a cathode.

The capping layer may be provided not only on the surface of the first electrode opposite to the organic layer but also on the surface of the second electrode opposite to the organic layer.

The first electrode disposed in contact with the capping layer is transparent or translucent. Therefore, the organic EL element has high light extraction efficiency. When the capping layer is also provided on the surface of the second electrode opposite to the organic layer, the second electrode disposed in contact with the capping layer is preferably transparent or translucent because it becomes an organic EL element with high light extraction efficiency. Transparent or translucent materials used for the first electrode and/or the second electrode include, for example, a MgAg alloy.

As the structure of the organic EL element of the present embodiment, for example, in the case of a light emitting element having a top emission structure, a multilayer structure in which an anode, a hole transport layer, a light emitting layer, an electron transport layer, a cathode, and a capping layer are sequentially provided on a glass substrate can be used. Further, a multilayer structure having a hole injection layer between the anode and the hole transport layer, a multilayer structure having an electron blocking layer between the hole transport layer and the light emitting layer, a multilayer structure having a hole blocking layer between the light emitting layer and the electron transport layer, or a multilayer structure having an electron injection layer between the electron transport layer and the cathode can also be used.

FIG. 1 is a schematic cross-sectional view showing an example of the organic EL element of this embodiment. The organic EL element shown in FIG. 1 has a top emission structure in which an anode 2, a hole injection layer 3, a hole transport layer 4, a light emitting layer 5, an electron transport layer 6, an electron injection layer 7, a cathode 8, and a capping layer 9 are laminated in this order on a glass substrate 1. In the organic EL element shown in FIG. 1, the first electrode is a cathode 8 and the second electrode is an anode. In the organic EL element shown in FIG. 1, the organic layer includes a hole transport layer 4, a light emitting layer 5, and an electron transport layer 6.

In these multilayer structures, several layers of the organic layer may be omitted or be used as a layer having two or more functions.

For example, a structure including a layer having both functions of a hole injection layer and a hole transport layer, a structure including a layer having both functions of a hole transport layer and an electron blocking layer, a structure including a layer having both functions of a hole blocking layer and an electron transport layer, or a structure including a layer having both functions of an electron transport layer and an electron injection layer may be used. It is also possible to form a structure including two or more organic layers having the same function. For example, a structure in which two hole transport layers are laminated, a structure in which two light emitting layers are laminated, a structure in which two electron transport layers are laminated, or a structure in which two capping layers are laminated can be used.

The total film thickness of each layer of the organic EL element is preferably about 200 nm to 750 nm, more preferably about 350 nm to 600 nm.

The capping layer 9 in the organic EL element shown in FIG. 1 contains a diamine compound represented by the above general formula (a-1) having a benzazole ring structure. In the organic EL element shown in FIG. 1, there are no particular restrictions on the materials used for the other layers than the capping layer 9. Hereinafter, the material of each layer of the organic EL element will be specifically described by way of example, but the material of each layer is not limited thereto.

### "Capping Layer"

In the organic EL element of this embodiment, the thickness of the capping layer is preferably in the range of 30 nm to 120 nm, and more preferably in the range of 40 nm to 80 nm. A thickness of the capping layer of 30 nm or more is preferable because the effect of having the capping layer becomes remarkable. When the thickness of the capping layer is 120 nm or less, it is preferable because the thickness of the capping layer can be suppressed from hindering the thinning of the organic EL element. When the film thickness of the capping layer is 30 nm to 120 nm, good light extraction efficiency is obtained.

The film thickness of the capping layer can be appropriately changed according to the type of light emitting material used for the light emitting layer and the thickness of each layer of the organic EL element other than the capping layer.

In the organic EL element of the present embodiment, the refractive index of the capping layer is preferably 1.85 or more, and more preferably 1.90 or more, when a light passing through the capping layer has a wavelength within the range of 450 nm to 750 nm.

The refractive index of the capping layer serves as an index for improving the light extraction efficiency of the organic EL element.

The refractive index of the capping layer is preferably larger than the refractive index of the adjacent electrodes. That is, the capping layer improves the light extraction efficiency of the organic EL element. The effect is effective because the larger the reflectance at the interface is between the capping layer and the material in contact with the capping layer, the larger the effect of the optical interference is. Therefore, the refractive index of the capping layer is preferably larger than the refractive index of the adjacent electrodes, and the refractive index of light having wavelengths of 400 nm and 410 nm is preferably 1.70 or more, more preferably 1.80 or more, and most preferably 1.85 or more.

The capping layer of the organic EL element of this embodiment preferably has a refractive index of 2.10 to 3.00 when light having wavelengths of 400 nm and 410 nm is transmitted. An organic EL element having a capping layer having a refractive index of 2.10 or more when light having wavelengths of 400 nm and 410 nm is transmitted is preferable because the organic EL element has high light extraction efficiency, high luminance, and good light emission efficiency and power efficiency.

The capping layer of the organic EL element of the present embodiment preferably has an extinction coefficient of 0.20 to 1.50 for light having wavelengths of 400 nm and 410 nm. The capping layer having an extinction coefficient of 0.20 or more for light having wavelengths of 400 nm and 410 nm has a good function of absorbing light having wavelengths of 400 nm and 410 nm. Therefore, the organic EL element having the capping layer having the extinction coefficient of 0.20 or more has good light resistance and can maintain color purity for a long period of time. As a result, the organic EL element has a long lifetime in which deterioration of luminance is suppressed.

The capping layer provided in the organic EL element of the present embodiment may be formed of only one thin film, or may be formed by laminating two or more thin films of different materials.

The capping layer may be formed of only one kind of material, or may be formed by mixing two or more kinds of materials.

The capping layer of the organic EL element of the present embodiment contains a diamine compound represented by the above general formula (a-1) having a benzazole ring structure.

The diamine compound can be formed into a film by a vapor deposition method. The diamine compound can be formed into a thin film by a known method such as a spin coating method and/or an ink jet method in addition to a vapor deposition method.

The capping layer may be formed by forming a film of the diamine compound alone, or a single layer formed by mixing the diamine compound with other materials. The capping layer may have a structure in which a plurality of layers formed by independently forming the diamine compound are laminated, a structure in which a plurality of layers formed by mixing the diamine compound with other materials are laminated, or a structure in which a layer formed by mixing the diamine compound with the diamine compound alone is laminated.

### "Compound Represented by General Formula (a-1)"

The capping layer provided in the organic EL element of the present embodiment contains a diamine compound represented by the above general formula (a-1) having a benzazole ring structure.

The diamine compound represented by general formula (a-1) has two amino group skeletons of NAr₁Ar₂ and NAr₃Ar₄. The diamine compound represented by general formula (a-1) has a benzoazole ring structure in which A is represented by formula (b-1). Alternatively, A and one or more of Ar₁ to Ar₄ have a benzazole ring structure represented by formula (b-1). Therefore, the diamine compound represented by the general formula (a-1) has a high absorbance of light having a wavelength of 400 nm to 410 nm, and can form a thin film having a high refractive index and a high extinction coefficient when light having wavelengths of 400 nm and 410 nm is transmitted. Therefore, the organic EL element of the present embodiment having the capping layer containing the diamine compound represented by formula (a-1) has high luminance, good light emission efficiency and power efficiency, and a long life.

Note that both of the two amino group skeletons (-NAr₁Ar₂ and NAr₃Ar₄) of the diamine compound represented by formula (a-1) are not amino groups represented by formula "-NH₂". NAr₁Ar₂ and NAr₃Ar₄ each have an amino group skeleton represented by "-NRR"' (R and R' indicate substituents) in which one hydrogen is removed from the secondary amine. The amino group skeleton represented by "-NRR"' of NAr₁Ar₂ and NAr₃Ar₄ may be one in which -N, and -R and/or -R' form a ring.

The term "aromatic hydrocarbon group", "aromatic heterocyclic group", or "condensed polycyclic aromatic group" in "substituted or unsubstituted aromatic hydrocarbon group", "substituted or unsubstituted aromatic heterocyclic group", or "substituted or unsubstituted fused polycyclic aromatic group" represented by Ar1 to Ar4 in general formula (a-1) are specifically selected from group consisted of a phenyl group, biphenylyl group, terphenylyl group, naphthyl group, anthracenyl group, phenanthrenyl group, fluorenyl group, spirobifluorenyl group, indenyl group, pyrenyl group, perylenyl group, fluoranthenyl group, triphenylenyl group, pyridyl group, pyrimidinyl group, triazinyl group, furyl group, furyl group, pyrrolyl group, thienyl group, quinolyl group, isoquinolyl group, benzofuranyl group, benzothienyl group, indyl group, carbazolyl group, benzoxazolyl group, benzoxazolyl group, pyrazolyl group, dibenzofuranyl group, dibenzothienyl group, and dibenzothienyl group. In addition to a naphthyridinyl group, a phenanthrolinyl group, an acridinyl group, and a carbonyl group, an aryl group having 6 to 30 carbon atoms and a heteroaryl group having 2 to 20 carbon atoms.

The term "substituent" in "substituted or unsubstituted aromatic hydrocarbon groups", "substituted or unsubstituted aromatic heterocyclic groups" or "substituted or unsubstituted fused polycyclic aromatic group" represented by Ar₁ to Ar₄ in general formula (a-1) refers specifically to a deuterium atom, trifluoromethyl group, cyano group, or nitro group; halogen atoms, such as fluorine, chlorine, bromine, and iodine atoms; silyl groups such as trimethylsilyl groups and triphenylsilyl groups; linear or branched alkyl group having 1 to 6 carbon atoms such as methyl group, ethyl group and propyl group; 1 to 6 carbon linear or branched alkyloxy group such as methyloxy, ethyloxy, or propyloxy; a aryloxy group such as a phenyloxy group or a tolyloxy group; arylalkyloxy groups such as benzyloxy and phenethyloxy groups; alkenyl groups such as vinyl and allyl groups; aralkyl groups such as benzyl, naphthylmethyl, and phenethyl groups; aromatic hydrocarbon groups or condensed polycyclic aromatic groups such as phenyl, biphenylyl, terphenylyl, naphthyl, anthracenyl, phenanthrenyl, fluorenyl, spirobifluorenyl, indenyl, pyrenyl, perylenyl, fluoranthenyl, and triphenylenyl groups; an aromatic heterocyclic group such as a pyridyl group, thienyl group, furyl group, pyrrolyl group, quinolyl group, isoquinolyl group, benzofuranyl group, benzothienyl group, indolyl group, carbazolyl group, benzoxazolyl group, benzothiazolyl group, quinoxalinyl group, benzimidazolyl group, pyrazolyl group, dibenzofuranyl group, dibenzothienyl group, carbolinyl group, aryl group having 6 to 30 carbon atoms, heteroaryl group having 2 to 20 carbon atoms, or the like. These substituents may be further substituted by the substituents exemplified above. Alternatively, a benzene ring substituted with these substituents or a plurality of substituents substituted with the same benzene ring may be bonded to each other via a single bond, a substituted or unsubstituted methylene group, a substituted or unsubstituted amino group, an oxygen atom, or a sulfur atom to form a ring.

Examples of "linear or branched alkyl group having 1 to 6 carbon atoms", "cycloalkyl group having 5 to 10 carbon atoms", and "linear or branched alkenyl group of 2 to 6 carbon atoms" in "linear or branched alkyl group of 1 to 6 carbon atoms which may have a substituent", "cycloalkyl group having 5 to 10 carbon atoms which may have a substituent", or "linear or branched alkenyl group of 2 to 6 carbon atoms which may have substituents", represented by R₁ to R₃₀ in formulas (a-1), (b-1), (b-2), (b-3), (b-4), (b-5), (b-6), (b-7), (b-8), (b-9), and (b-10), include methyl group, ethyl group, n-propyl group, isopropyl group, n-butyl group, isobutyl group, tert-butyl group, n-pentyl group, isopentyl group, neopentyl group, n-hexyl group, cyclopentyl group, cyclohexyl group, 1-adamantyl group, vinyl group, allyl group, isopropenyl group, 2-butenyl group, and the like. These groups may be bonded together to the same substituted benzene ring via a single bond, a substituted or unsubstituted methylene group, a substituted or unsubstituted amino group, an oxygen atom, or a sulfur atom to form a ring.

Examples of " linear or branched alkyloxy group having 1 to 6 carbon atoms", "cycloalkyloxy group having 5 to 10 carbon atoms", and "aryloxy group" in "linear or branched alkyloxy group having 1 to 6 carbon atoms which may have a substituent", "cycloalkyloxy group having 5 to 10 carbon atoms which may have a substituent", or "substituted or unsubstituted aryloxy group", represented by R₁ to R₃₀ in general formulas (a-1), (b-1), (b-2), (b-3), (b-4), (b-5), (b-6), (b-7), (b-8), (b-9), and (b-10), include methyloxy group, ethyloxy group, n-propyloxy group, cyclopentyloxy group, cyclohexyloxy group, 1-adamantyloxy group, 2-adamantyloxy group, phenyloxy group, tolyloxy group, naphthyloxy group, fluorenyloxy group, and biphenyloxy group. These groups may be bonded together to the same substituted benzene ring via a single bond, a substituted or unsubstituted methylene group, a substituted or unsubstituted amino group, an oxygen atom, or a sulfur atom to form a ring.

Examples of "aromatic hydrocarbon group", "aromatic heterocyclic group", and "condensed polycyclic aromatic group" in "Substituted or unsubstituted aromatic hydrocarbon groups", "substituted or unsubstituted aromatic heterocyclic groups", or "substituted or unsubstituted fused polycyclic aromatic group", represented by R₁ to R₃₀ in the general formulas (a-1), (b-1), (b-2), (b-3), (b-4), (b-5), (b-6), (b-7), (b-8), (b-9), and (b-10), may be the same as those shown in relation to "aromatic hydrocarbon group", "aromatic heterocyclic group", or "condensed polycyclic aromatic group" in "substituted or unsubstituted aromatic hydrocarbon groups", "substituted or unsubstituted aromatic heterocyclic groups", or "substituted or unsubstituted fused polycyclic aromatic group", represented by Ar₁ to Ar₄ in the general formula (a-1), and can be similar in possible embodiments.

Examples of "substituent" in "linear or branched alkyl group of 1 to 6 carbon atoms which may have a substituent", "cycloalkyl group having 5 to 10 carbon atoms which may have a substituent", "linear or branched alkenyl group of 2 to 6 carbon atoms which may have substituents", "linear or branched alkyloxy group having 1 to 6 carbon atoms which may have a substituent", "cycloalkyloxy group having 5 to 10 carbon atoms which may have a substituent", "substituted aryloxy group", "substituted or unsubstituted aromatic hydrocarbon groups", "substituted or unsubstituted aromatic heterocyclic groups", or "substituted or unsubstituted fused polycyclic aromatic group", represented by R₁ to R₃₀ in the general formulas (a-1), (b-1), (b-2), (b-3), (b-4), (b-5), (b-6), (b-7), (b-8), (b-9), and (b-10), include those similar to those shown for "substituent" in "substituted or unsubstituted aromatic hydrocarbon groups", "substituted or unsubstituted aromatic heterocyclic groups", or "substituted or unsubstituted fused polycyclic aromatic group", represented by Ar₁ to Ar₄ in the above general formula (a-1), and can include similar embodiments.

As the diamine compound represented by general formula (a-1), the two amino group skeletons of -NAr₁Ar₂ and NAr₃Ar₄ in the general formula (a-1) may be the same or different. When the two amino group skeletons of -NAr₁Ar₂ and NAr₃Ar₄ are the same, it is preferable because the synthesis is easy.

At least two of Ar₁ to Ar₄ in the above general formula (a-1) are preferably substituted or unsubstituted aromatic hydrocarbon groups, and more preferably substituted or unsubstituted phenyl or biphenyl groups.

It is preferable that all of the substituents Ar₁ to Ar₄ in the two amino group skeletons (-NAr₁Ar₂ and NAr₃Ar₄) included in the general formula (a-1) are unsubstituted phenyl groups or biphenyl groups.

The sum of m and n in the general formula (a-1) is preferably 2 or less (0, 1, or 2), more preferably 1 or less (0 or 1), and most preferably both m and n are 0.

In the diamine compound represented by general formula (a-1), X in the benzazole ring structure represented by formula (b-1) as A is preferably carbon or nitrogen, and Y is preferably oxygen or nitrogen. Further, when X is nitrogen, it is more preferable that Y is also nitrogen. When X is carbon, it is more preferable that Y is oxygen. When X in formula (b-1) as A in formula (a-1) is carbon and Y is oxygen, the extinction coefficient is 0.2 or more, and the absorbance is large, so that the organic EL element having the capping layer containing this compound is more improved in light extraction efficiency.

Among the diamine compounds represented by formula (a-1) having a benzazole ring structure and suitably used for the organic EL element of this embodiment, compounds represented by formulas (1) to (103) are shown below as specific examples of preferred compounds. The compound represented by formula (a-1) is not limited to the compounds represented by formulas (1) to (103).

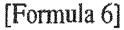

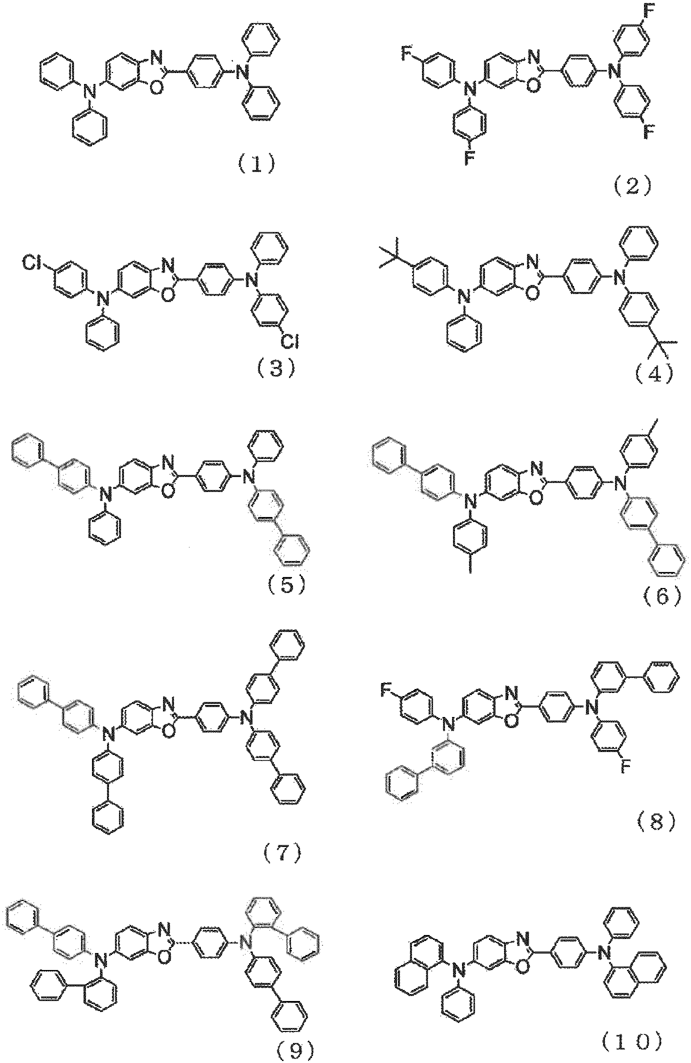

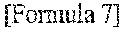

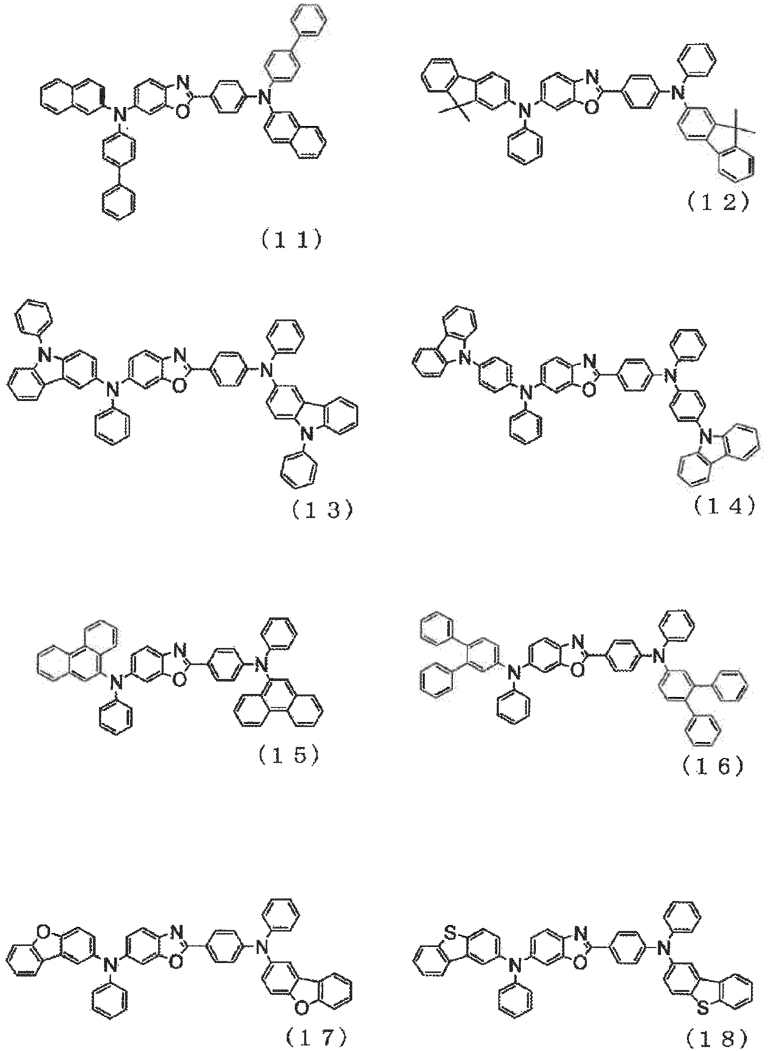

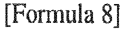

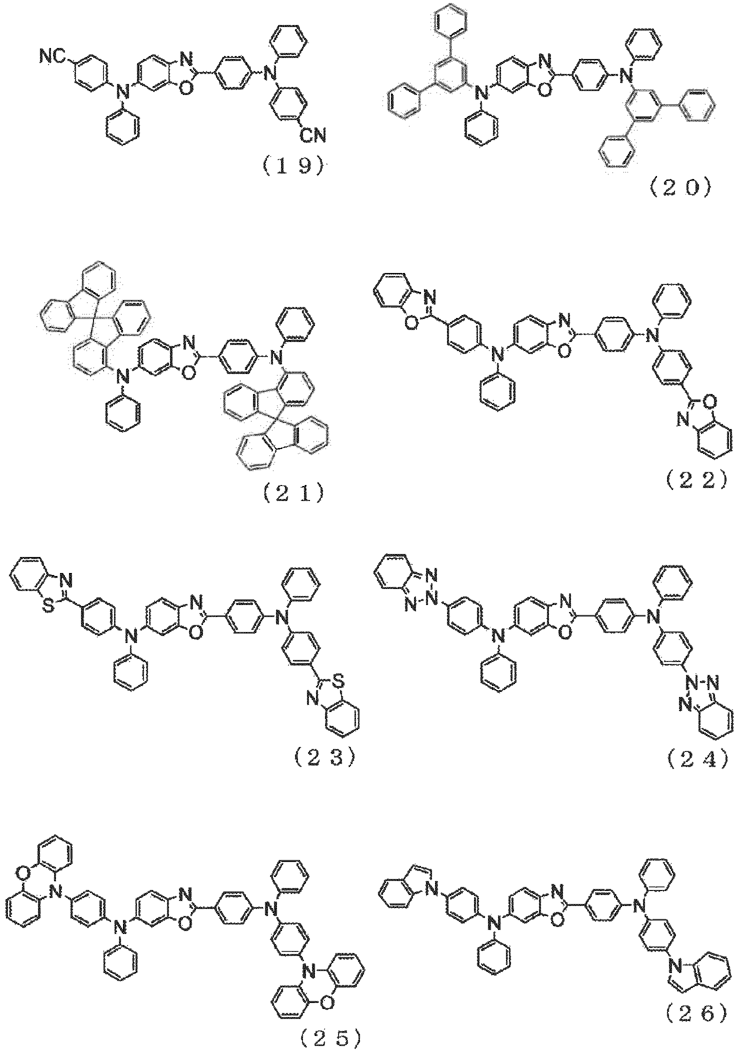

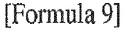

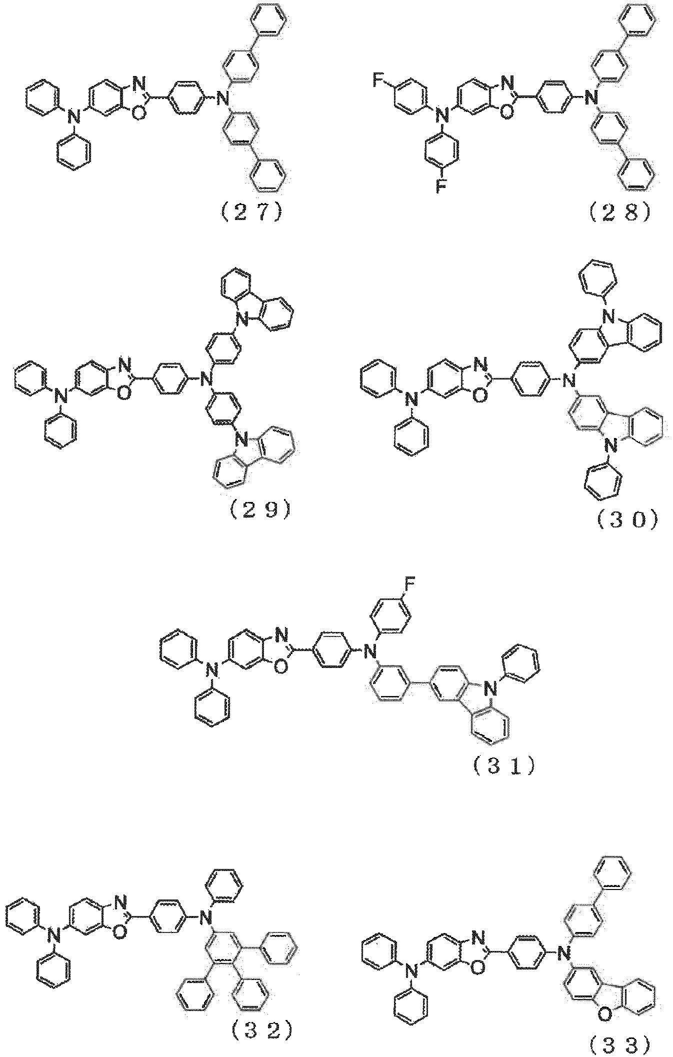

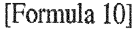

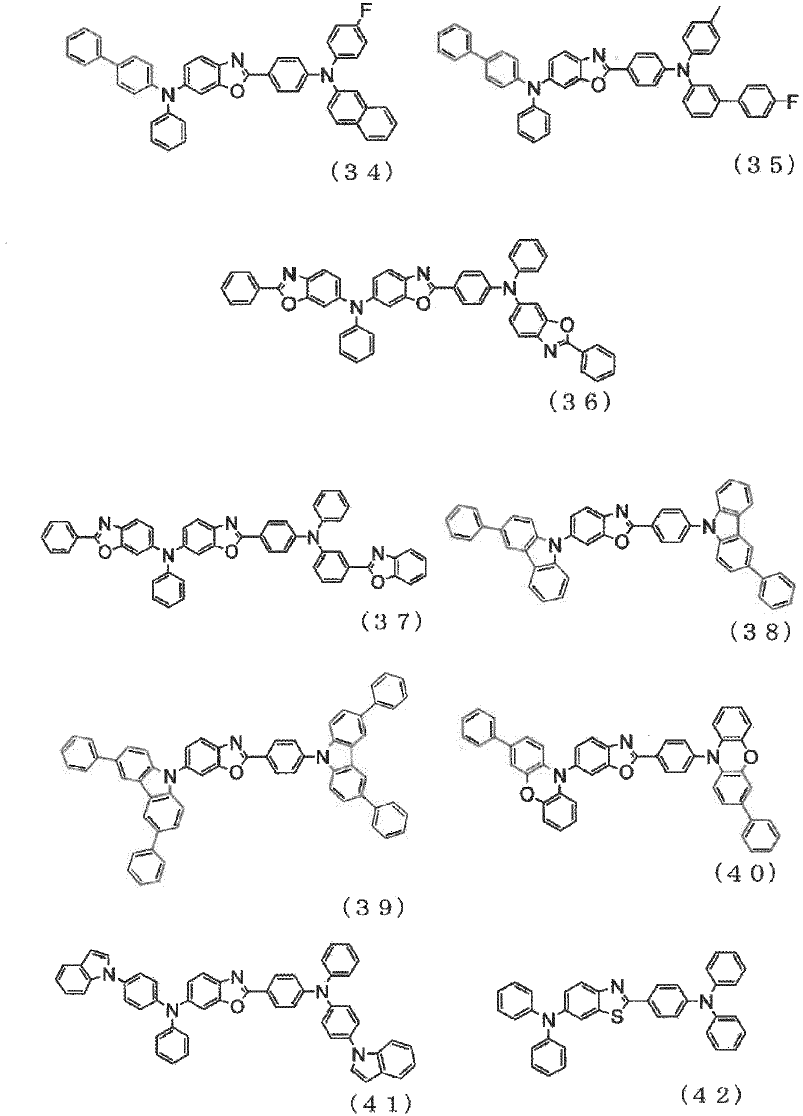

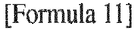

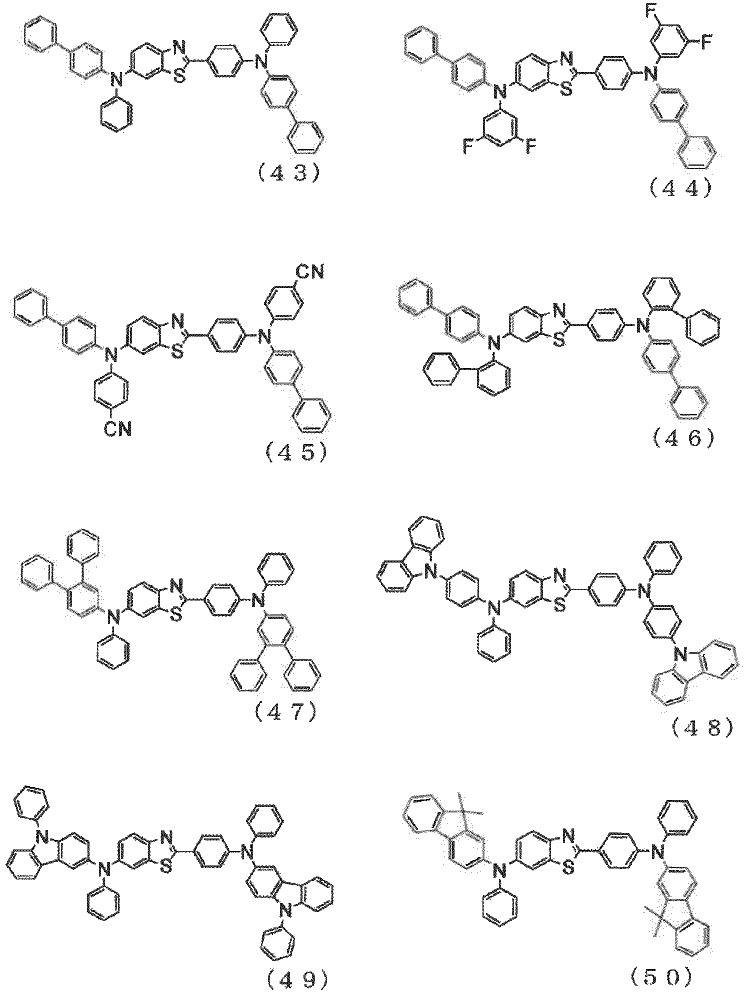

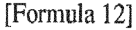

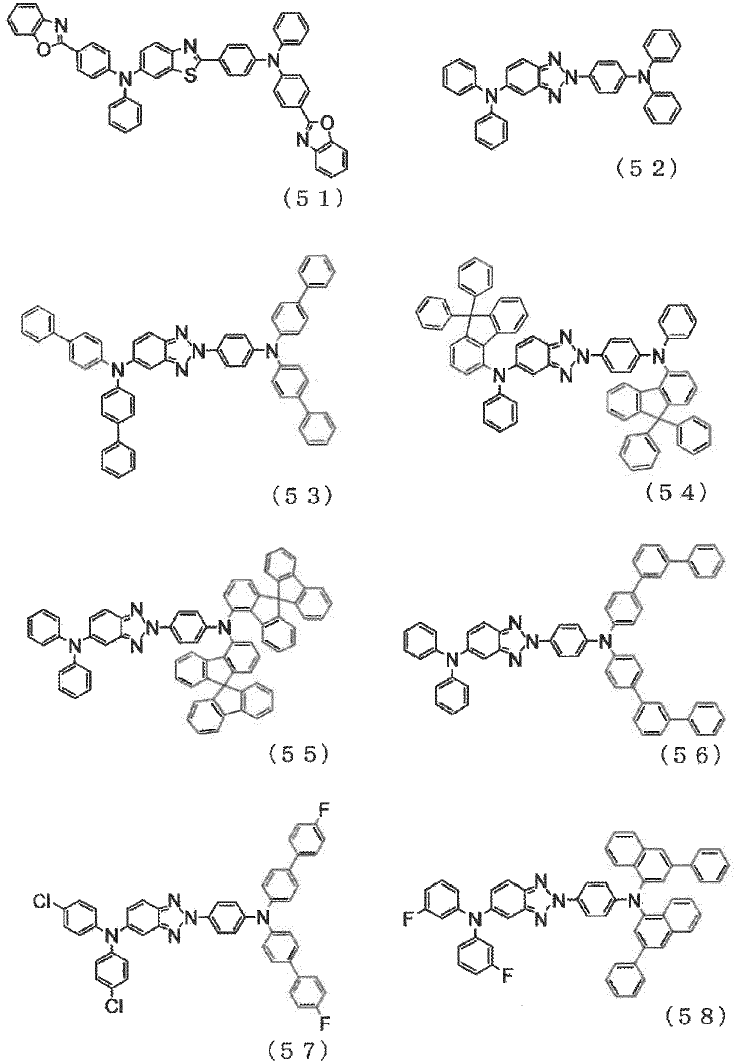

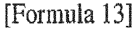

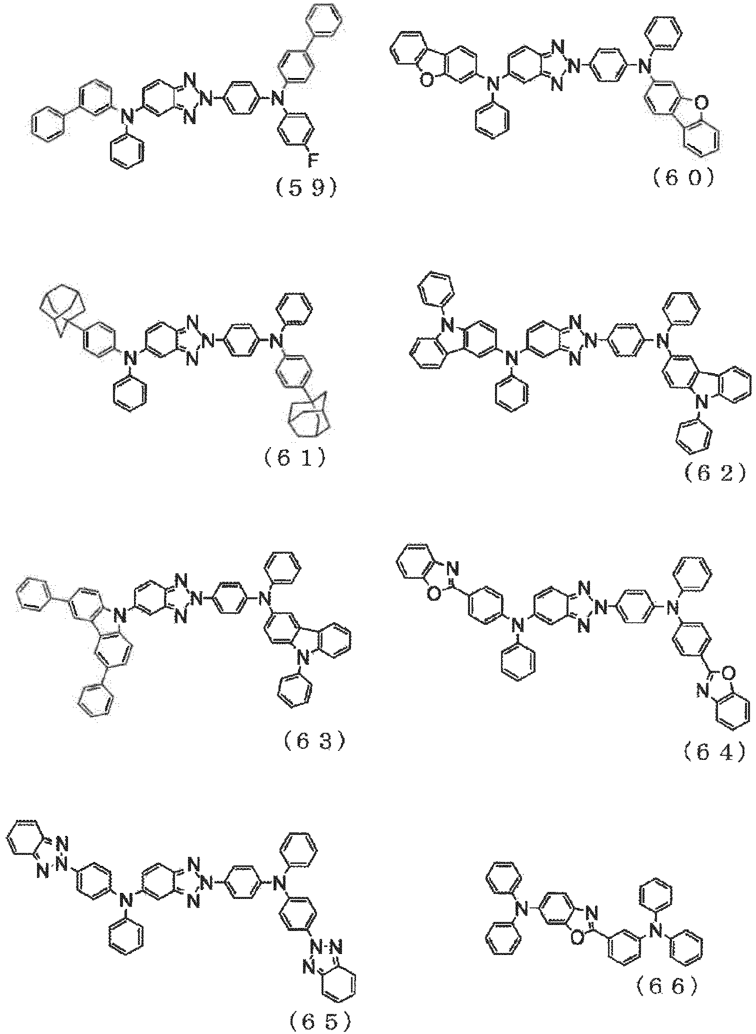

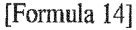

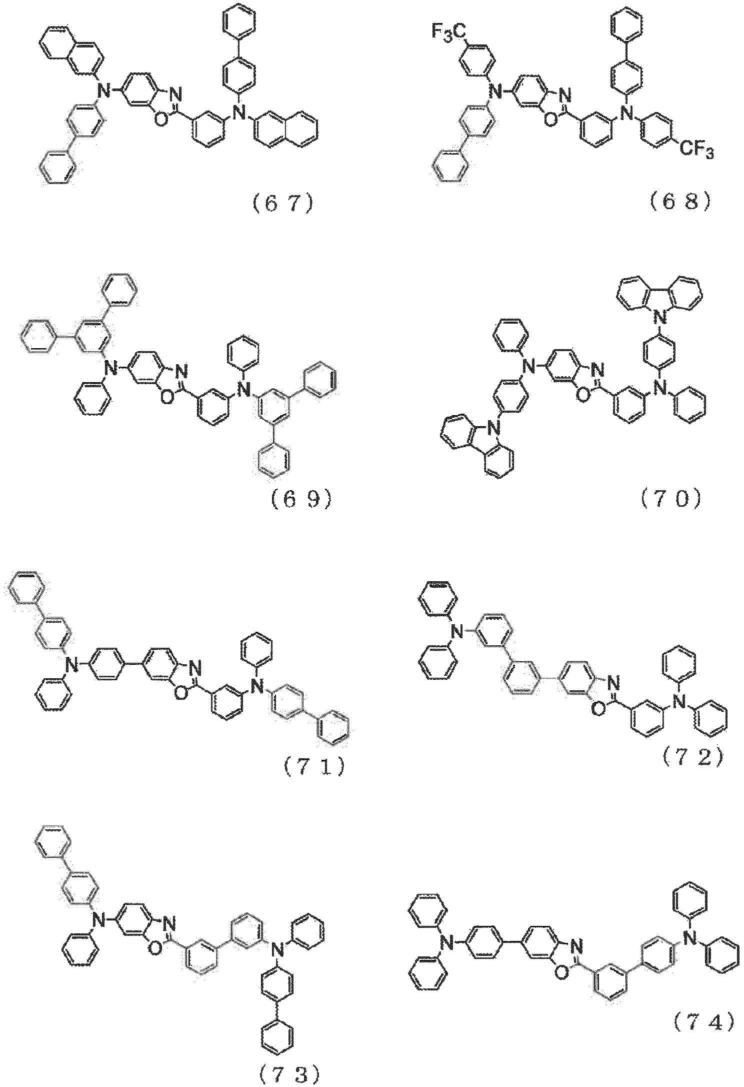

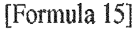

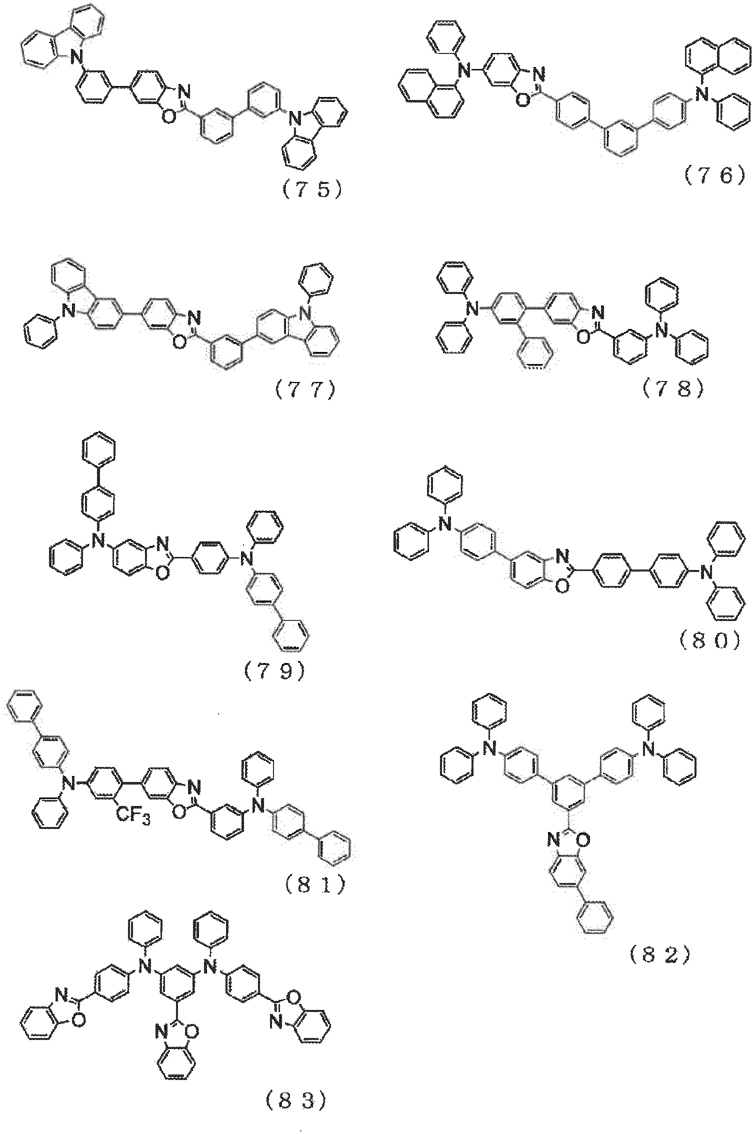

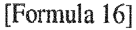

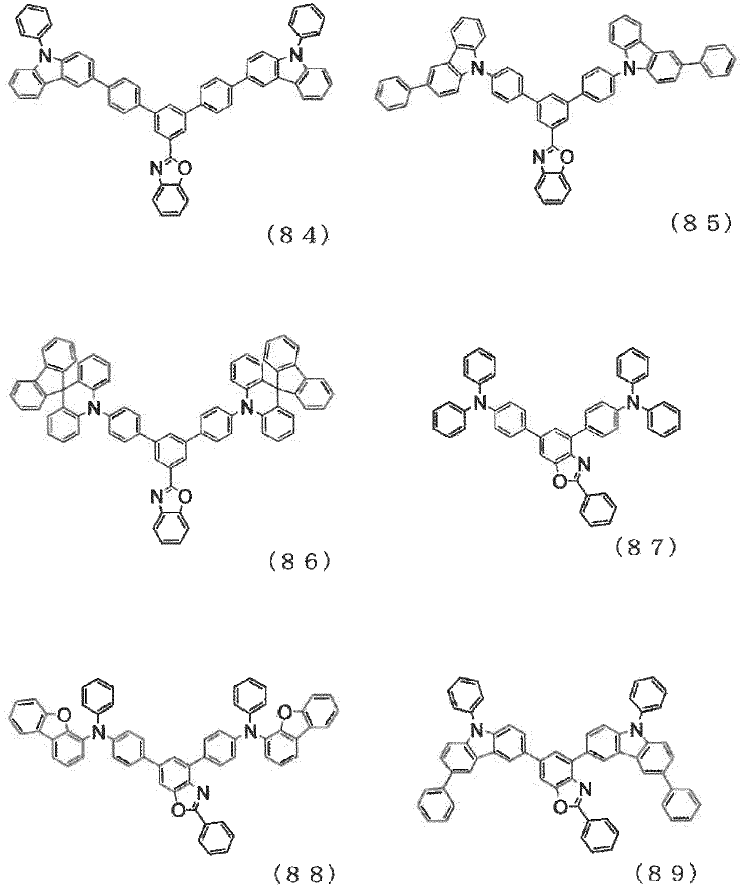

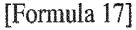

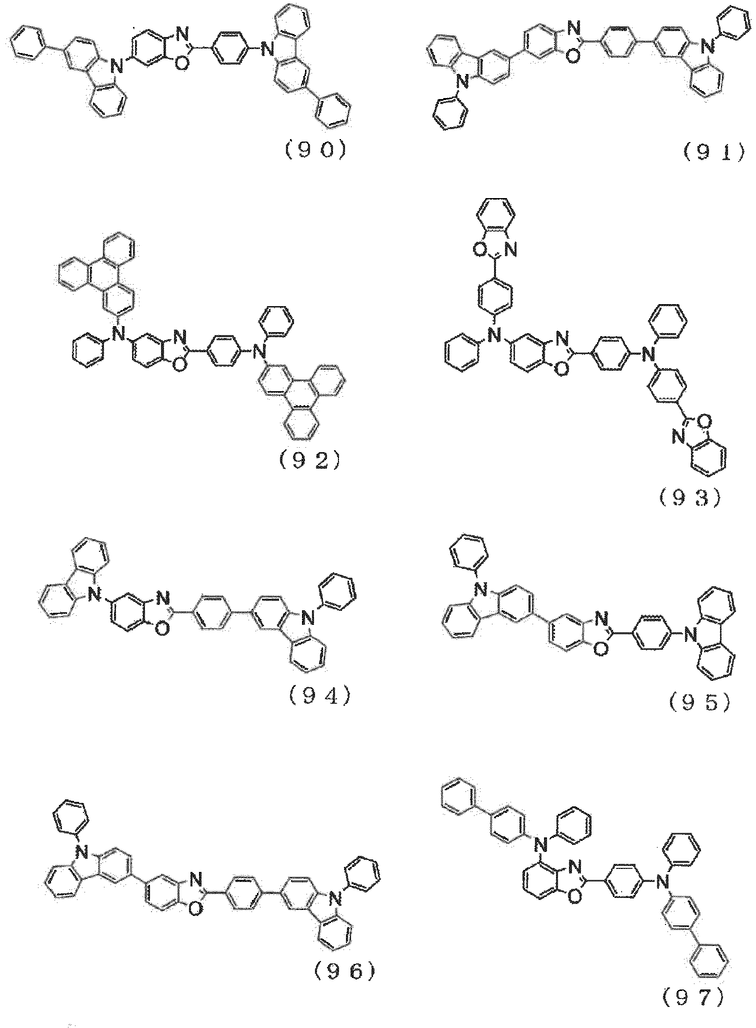

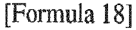

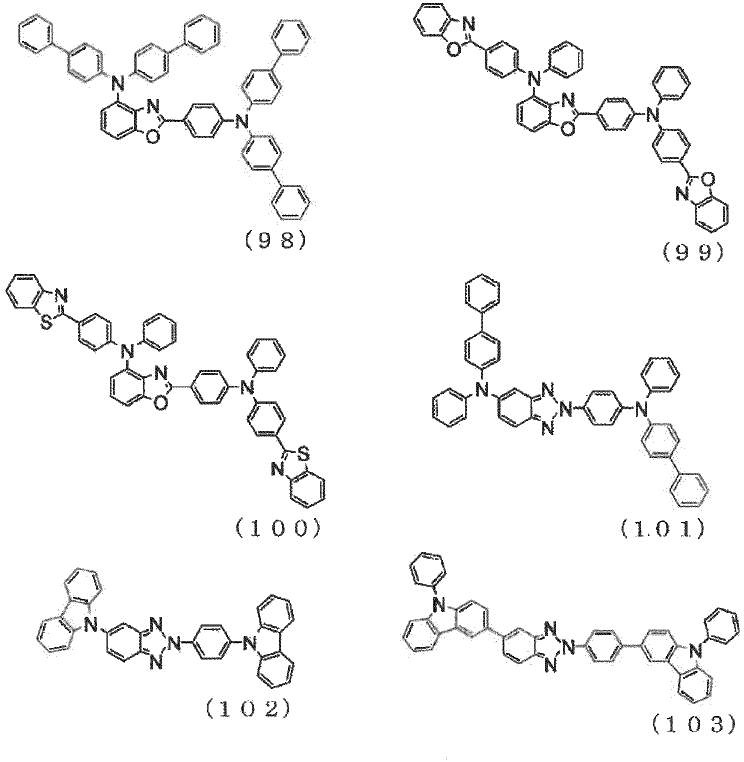

As the material of the capping layer, it is preferable to use one or more compounds selected from compounds represented by formula (7), (22), (79), (93), (97), (99), (101), and (102) among compounds represented by formulas (1) to (103).

These compounds have an absorbance of 0.2 or more between 400 nm and 410 nm at a concentration of 10⁻⁵ mol/L, and have a good function of absorbing light between 400 nm and 410 nm.

The deposited film formed by depositing these compounds and having a thickness of 30 nm to 120 nm has a refractive index of 2.10 to 3.00 and an extinction coefficient of 0.20 to 1.50 when light having wavelengths of 400 nm and 410 nm is transmitted.

Therefore, by using one or more compounds selected from compounds represented by formula (7), (22), (79), (93), (97), (99), (101), and (102) as a material for the capping layer, it is possible to obtain an organic EL element having higher luminance, better luminous efficiency and power efficiency, and a longer life.

The compound represented by general formula (a-1) preferably has a glass transition point (Tg) of 100°C or higher. The glass transition point (Tg) of the compound is an index of the stability of the thin film state. When the glass transition point (Tg) of the compound is 100°C or higher, a thin film having good stability can be formed, and the thin film is preferable as a material for the capping layer. An organic EL element having a capping layer with good stability has a long lifetime and is preferable.

The melting point and the glass transition point (Tg) of the compound represented by formula (a-1) in the present embodiment are obtained by measuring with a highly sensitive differential scanning calorimeter (manufactured by Bruker AXS, DSC3100SA) using a powder of the compound.

The compound represented by general formula (a-1) preferably has an absorbance of 0.2 to 1.5 between 400 nm and 410 nm at a concentration of 10⁻⁵ mol/L, and more preferably has an absorbance of 0.6 to 1.5 at 400 nm. A capping layer containing a compound having an absorbance of 0.2 or more between 400 nm and 410 nm has a good function of absorbing light at wavelengths of 400 nm and 410 nm. Therefore, an organic EL element having a capping layer containing a compound having an absorbance of 0.2 or more has good light resistance and a long life.

The absorbance of the compound represented by general formula (a-1) was measured using an ultraviolet-visible near-infrared spectrophotometer (manufactured by JASCO Co., Ltd., V -650) after adjusting the concentration to 10⁻⁵ mol/L in a toluene solvent.

The compound represented by formula (a-1) preferably has a light absorption coefficient in the range of 80000 to 200000. When the light absorption coefficient of the compound is within the above range, a thin film having a good function of absorbing light can be formed, and the thin film is preferable as a material for the capping layer.

The absorption coefficient of the compound represented by formula (a-1) was determined by the following method. First, a sample of a compound adjusted to four concentrations of 5.0×10⁻⁶mol/L, 1.0×10⁻⁵ mol/L, 1.5×10⁻⁵ mol/L, 2.0×10⁻⁵ mol/L in toluene solution is prepared. Next, the absorbance of each sample at the peak wavelength is measured using an ultraviolet-visible near-infrared spectrophotometer (manufactured by JASCO Co., Ltd., V-650). A calibration curve is prepared using the results, and the absorption coefficient of the compound is calculated.

### "Method for Producing Compound Represented by General Formula (a-1)"

The diamine compound represented by general formula (a-1) having a benzazole ring structure can be produced, for example, by the following method.

First, a halogenated benzoazole derivative used as a raw material for forming a benzoazole ring structure represented by formula (b-1) is produced. For example, the halogenated benzazole derivative having a structure corresponding to the benzazole ring structure represented by formula (b-1) can be synthesized by a method known, (for example, see Non-Patent Documents 4 and 5).

In the following reaction formulas, X, X' and X" are all halogens. Further, -N(Ar')2 and N(Ar")₂ are -NAr₁Ar₂ or NAr₃Ar₄ in the general formula (a-1).

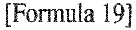

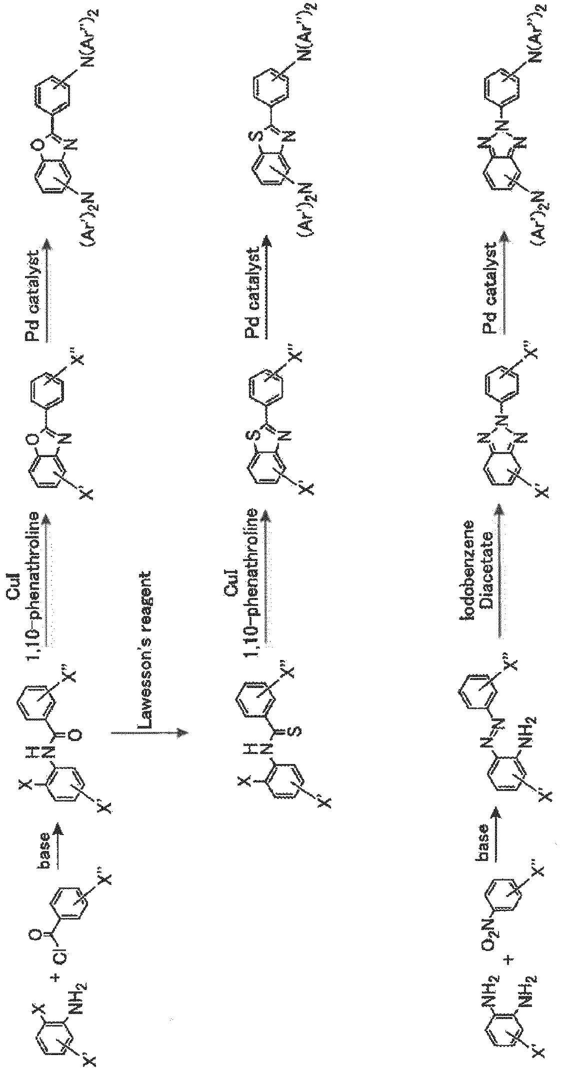

Further, the synthesized halogenated benzazole derivative is coupled with, for example, an arylamine by using a copper catalyst and/or a palladium catalyst. Thus, a diamine compound represented by general formula (a-1) having a benzazole ring structure can be synthesized.

A diamine compound represented by general formula (a-1) having a benzoazole ring structure can be similarly synthesized by a coupling reaction with a halogenated arylamine by using a boronic acid derivative or a boronic ester derivative instead of the halogenated benzoazole derivative (for example, see Non-Patent Documents 6 and 7).

The compound represented by general formula (a-1) thus synthesized is preferably used after purification by column chromatography, adsorption purification by silica gel, activated carbon, activated clay, or the like, recrystallization by a solvent, crystallization, or sublimation purification.

The compound represented by formula (a-1) thus synthesized can be identified by nuclear magnetic resonance (NMR) analysis.

### "Anode"

In the organic EL element of this embodiment, an anode is provided on a glass substrate. As the material of the anode, an electrode material having a large work function such as ITO (indium tin oxide) and gold is used.

As a method of manufacturing the anode, a known method such as a vapor deposition method can be used.

### "Organic Layer"

In the present embodiment, the organic layer including a hole injection layer, a hole transport layer, an electron blocking layer, a light emitting layer, a hole blocking layer, an electron transport layer, and an electron injection layer stacked in this order from the anode side will be described as an example.

### (Hole Injection Layer)

As the material of the hole injection layer of the organic EL element of this embodiment, an arylamine compound having a structure in which three or more triphenylamine structures are connected by a divalent group containing no single bond or heteroatom in the molecule, for example, a material such as a starburst type triphenylamine derivative, various triphenylamine tetramers, a porphyrin compound represented by copper phthalocyanine, an acceptor heterocyclic compound such as hexacyanoazatriphenylene, or a coating type polymer material can be used.

As the hole injection layer, a single layer formed by mixing two or more kinds of materials may be used. The hole injection layer may have a structure in which a plurality of layers formed by independently forming the above material are laminated, a structure in which a plurality of layers formed by mixing the above material with another material are laminated, or a structure in which a layer formed by mixing the above material with a layer formed by independently forming a film is laminated.

These materials may be formed into thin films by a vapor deposition method or by a known method such as a spin coating method or an ink jet method in addition to the vapor deposition method.

### (Hole Transport Layer)

As the material of the hole transport layer of the organic EL element of this embodiment, benzidine derivatives such as N,N'-diphenyl-N,N'-di(m-tolyl)benzidine (hereinafter referred to as TPD), N,N'-diphenyl- N,N'-di(alpha-naphthyl) benzidine (hereinafter referred to as NPD), N,N,N',N'-tetrabiphenylbenzidine; 1,1-bis[4-(di-4-tolylamino)phenyl]cyclohexane (hereinafter referred to as TAPC), or the like is preferably used. As the material of the hole transport layer, it is particularly preferable to use an arylamine compound having a structure in which two triphenylamine structures are connected by a divalent group containing no single bond or heteroatom in the molecule, for example, N,N,N',N'-tetrabiphenylylbenzidine or the like. As the material of the hole transport layer, it is preferable to use an arylamine compound having a structure in which three or more triphenylamine structures are connected by a divalent group containing no single bond or heteroatom in the molecule, for example, various triphenylamine trimers and tetramers.

As the hole transport layer, a single layer formed by mixing two or more kinds of materials may be used. The hole transport layer may have a structure in which a plurality of layers formed of the above material alone are laminated, a structure in which a plurality of layers formed by mixing with other materials are laminated, or a structure in which a layer formed by mixing with a layer formed alone is laminated.

These materials may be formed into thin films by a vapor deposition method or by a known method such as a spin coating method or an ink jet method in addition to the vapor deposition method.

As the material of the hole injection layer and the hole transport layer, a coating type polymer material such as poly (3,4-Ethylenedioxythiophene) (hereafter abbreviated as PEDOT)/poly (styrene sulfonate) (hereinafter referred to as PSS) may be used.

In the hole injection layer or the hole transport layer, a material obtained by further P doping a material usually used for the layer, with trisbromophenylamine hexachlorantimony, a radialene derivative (for example, see Patent Document 3), or the like, or a polymer compound having a structure of a benzidine derivative such as TPD in its partial structure may be used.

### (Electron Blocking Layer)

As a material of the electron blocking layer of the organic EL element of the present embodiment, a compound having an electron blocking function such as a carbazole derivative such as 4,4',4"-tri(N-carbazolyl) triphenylamine (hereinafter referred to as TCTA), 9,9-bis[4-(carbazole-9-yl)phenyl]fluorene, 1,3-bis(carbazole-9-yl)benzene (hereinafter referred to as mCP), 2,2-bis(4-carbazole-9-yl-phenyl)adamantane (hereafter abbreviated as Ad-Cz); and a compound having a triphenylsilyl group and a triarylamine structure represented by 9-[4-(carbazole-9-yl)phenyl]-9-[4-(triphenylsilyl)phenyl]-9H-fluorene can be used.

As the electron blocking layer, a single layer formed by mixing two or more kinds of materials may be used. The electron blocking layer may have a structure in which a plurality of layers formed by independently forming a film of the above material are laminated, a structure in which a plurality of layers formed by mixing with other materials are laminated, or a structure in which a layer formed by mixing with a layer formed by independently forming a film is laminated.

These materials may be formed into thin films by a vapor deposition method or by a known method such as a spin coating method or an ink jet method in addition to the vapor deposition method.

### (Light Emitting Layer)

As a material of the light emitting layer of the organic EL element of this embodiment, in addition to metal complexes of quinolinol derivatives such as Alq₃, various metal complexes, anthracene derivatives, bisstyrylbenzene derivatives, pyrene derivatives, oxazole derivatives, polyparaphenylenevinylene derivatives, and the like can be used.

The light emitting layer may be composed of a host material and a dopant material. As the host material, an anthracene derivative is preferably used. As the host material, in addition to the light emitting material, a heterocyclic compound having an indole ring as a partial structure of a condensed ring, a heterocyclic compound having a carbazole ring as a partial structure of the condensed ring, a carbazole derivative, a thiazole derivative, a benzimidazole derivative, a polydialkylfluorene derivative, or the like can be used. As the dopant material, quinacridone, coumarin, rubrene, perylene, and their derivatives; benzopyran derivatives; rhodamine derivatives; aminostyryl derivatives; and the like can be used.

As the light emitting layer, a single layer formed by mixing two or more kinds of materials may be used. The light emitting layer may have a structure in which a plurality of layers formed by independently forming the above material are laminated, a structure in which a plurality of layers formed by mixing the above material with another material are laminated, or a structure in which a layer formed by mixing the above material with a layer formed by independently forming the film is laminated.

It is also possible to use a phosphorescent emitter as the light emitting material. As the phosphorescent emitter, a phosphorescent emitter of a metal complex such as iridium or platinum can be used. For example, a green phosphorescent emitter such as Ir(ppy)₃; a blue phosphorescent emitter such as bis(3,5-difluoro-2-(2-carboxypyridyl) iridium (III) (FIrpic), bis(2,4-difluorophenylpyridinato)-tetrakis(1-pyrazolyl) borate iridium (III) (FIr6); a red phosphorescent emitter such as bis(2-benzo[b]thiophene-2yl-pyridine) (acetylacetonate) iridium (III) (Btp₂Ir(acac)); and the like can be used.

As the host material at this time, carbazole derivatives such as 4,4'-di (N-carbazolyl) biphenyl (hereafter referred to as CBP), TCTA, and mCP can be used as the host material having hole injection/transport properties. A p-bis(triphenylsilyl) benzene (hereinafter referred to as UGH2), 2,2',2"-(1,3,5-phenylene)-tris(1-phenyl-1H-benzimidazole) (hereafter referred to as TPBI), or the like can be used as the host material of the electron transport property, and a high-performance organic EL element can be produced.

Doping of the phosphorescent luminescent material into the host material is preferably co-evaporated in a range of 1 to 30 weight percent relative to the entire luminescent layer to avoid concentration quenching.

It is also possible to use, as the light emitting material, a material emitting delayed fluorescence such as 2-biphenyl-4,6-bis(12-phenylindolo[2,3-a]carbazole-11-yl)-1,3,5-triazine (PIC-TRZ), 2,4-bis(f3-(9H-carbazole-9-yl)-6-phenyl-1,3,5-triazine (CC2TA), 2,4,6-tri(4-(10H-phenoxazine-10H-yl)phenyl)-1,3,5-triazine (PXZ-TRZ), 2,4,5,6-tetra(9H-carbazole-9-yl)isophthalonitrile (4CyIPN), carbazolyldicyanobenzene (CDCB) derivatives, and the like (for example, see Non-Patent Document 8).

These light emitting materials may be formed into thin films by a vapor deposition method, or may be formed into thin films by a known method such as a spin coating method or an ink jet method in addition to the vapor deposition method.

### (Hole Blocking Layer)

As a material of the hole blocking layer of the organic EL element of this embodiment, a compound having a hole blocking function such as a phenanthroline derivative such as bathocuproine (hereinafter abbreviated as BCP), a metal complex of a quinolinol derivative such as aluminum (III) bis(2-Methyl-8-quinolinate)-4-phenylphenolate (hereinafter abbreviated as BAlq), various rare earth complexes, triazole derivatives, triazine derivatives, pyrimidine derivatives, oxadiazole derivatives, benzazole derivatives, etc., can be used. These materials may also serve as materials for the electron transport layer.

As the hole blocking layer, a single layer formed by mixing two or more kinds of materials may be used. The hole blocking layer may have a structure in which a plurality of layers formed by independently forming the above material are laminated, a structure in which a plurality of layers formed by mixing the above material with another material are laminated, or a structure in which a layer formed by mixing the above material with a layer formed by independently forming a film is laminated.

These materials may be formed into thin films by a vapor deposition method or by a known method such as a spin coating method or an ink jet method in addition to the vapor deposition method.

### (Electron Transport Layer)

As a material of the electron transport layer of the organic EL element of this embodiment, in addition to metal complexes of quinolinol derivatives such as Alq₃ and BAlq, various metal complexes, triazole derivatives, triazine derivatives, pyrimidine derivatives, oxadiazole derivatives, pyridine derivatives, benzimidazole derivatives, benzazole derivatives, thiadiazole derivatives, anthracene derivatives, carbodiimide derivatives, quinoxaline derivatives, pyridoindole derivatives, phenanthroline derivatives, silol derivatives, and the like can be used.

As the electron transport layer, a single layer formed by mixing two or more kinds of materials may be used. The electron transport layer may have a structure in which a plurality of layers formed by independently forming a film of the above material are laminated, a structure in which a plurality of layers formed by mixing with other materials are laminated, or a structure in which a layer formed by mixing with a layer formed by independently forming a film is laminated.

These materials may be formed into thin films by a vapor deposition method or by a known method such as a spin coating method or an ink jet method in addition to the vapor deposition method.

### (Electron Injection Layer)

As a material of the electron injection layer of the organic EL element of this embodiment, an alkali metal salt such as lithium fluoride and cesium fluoride, an alkaline earth metal salt such as magnesium fluoride, a metal complex of a quinolinol derivative such as lithium quinolinol, a metal oxide such as aluminum oxide, or a metal such as ytterbium (Yb), samarium (Sm), calcium (Ca), strontium (Sr), cesium (Cs), or the like can be used.

The electron injection layer may be omitted in the preferred selection of the electron transport layer and the cathode.

Further, in the electron injection layer or the electron transport layer, it is possible to use a material which is normally used for the layer and is further doped with a metal such as cesium by N doping.

As a method of manufacturing the electron injection layer, a known method such as a vapor deposition method can be used.

### "Cathode"

As the material of the cathode of the organic EL element of this embodiment, an electrode material having a low work function such as aluminum, an alloy having a lower work function such as a magnesium-silver alloy, a magnesium-calcium alloy, a magnesium-indium alloy, an aluminum-magnesium alloy, or a transparent electrode material (IZO) made of ITO, indium oxide, and zinc oxide is used.

In the organic EL element of this embodiment, the first electrode disposed in contact with the capping layer is a cathode. Therefore, the cathode is preferably transparent or translucent in order to make an organic EL element having high light extraction efficiency.

As the manufacturing method of the cathode, a known method such as a vapor deposition method can be used.

The organic EL element of this embodiment has a top emission structure and has a capping layer provided outside the transparent or translucent electrode and having a refractive index higher than that of the translucent electrode. Thus, in the organic EL element of this embodiment, the light extraction efficiency can be greatly improved. The film can be formed at ≤ 400°C by using a diamine compound having a benzazole ring structure represented by the general formula (a-1) for the capping layer. Therefore, the capping layer in which the light extraction efficiency of each color is optimized can be formed by using the high-definition mask without damaging the light emitting element. Therefore, the organic EL element of the present embodiment can be suitably applied to a full-color display, and can display a clear and bright image with good color purity.

In the organic EL element of the present embodiment, a material for the organic EL element having a high absorption coefficient, a high refractive index, and excellent stability, durability, and light resistance of a thin film is used as a material for the capping layer. Therefore, the organic EL element of the present embodiment is not affected by sunlight, retains color purity, and can greatly improve the light extraction efficiency as compared with the conventional organic EL element. Furthermore, the organic EL element of the present embodiment has made it possible to realize an organic EL element having a high efficiency and a long life.

Although the organic EL element having the top emission structure has been described above, the present invention is not limited thereto. The present invention can be similarly applied to an organic EL element having a bottom emission structure and an organic EL element having a dual emission structure which emits light from both the top and the bottom. In the case of the organic EL element having these structures, the electrode in the direction in which light is extracted from the light emitting element is preferably transparent or translucent.

### "Manufacturing Method of Organic EL Element"

The method of manufacturing an organic EL element of the present embodiment includes a step of forming an organic layer including a light emitting layer between a first electrode and a second electrode, and a step of laminating a capping layer on a surface of the first electrode opposite to the organic layer.

In this embodiment, the capping layer is formed by using a diamine compound having a benzazole ring structure and represented by general formula (a-1). As a method for laminating the capping layer, it is preferable to use a vapor deposition method because the method is suitable for mass-producing a thin film having a thickness of nano units.

Hereinafter, embodiments of the present invention will be specifically described with reference to examples. The present invention is not limited to the following examples unless the gist thereof is exceeded.

### "Example 1"

### Synthesis of a compound represented by formula (7) <6-(bis-biphenyl-4-yl-amino)-2-{4-(bis-biphenyl-4-yl-amino)-phenyl}benzoxazole>

To a nitrogen-substituted reaction vessel was added 7.0 g of 6-bromo-2-(4-bromophenyl)-benzoxazole, 14.0 g of bis-(biphenyl-4-yl)-amine, 5.7 g of sodium t-butoxy, and 140 mL of toluene, and the vessel was aerated with nitrogen gas under ultrasonic irradiation for 30 minutes. To the reaction vessel was further added 0.2 g of palladium acetate and 0.4 g of tri(tert-butyl) phosphine, and the mixture was stirred under heated reflux for 3 hours. The reaction vessel was allowed to cool, and then filtered and concentrated under reduced pressure to give a crude product.

The resulting crude product was purified by re-crystallization in a toluene/acetone mixed solvent to obtain 15.0 g (yield of 91%) of a yellow powder of 6-(bis-biphenyl-4-yl-amino)-2-(4-(bis-biphenyl-4-yl-amino)-phenyl}-benzoxazole (Compound represented by formula (7)).

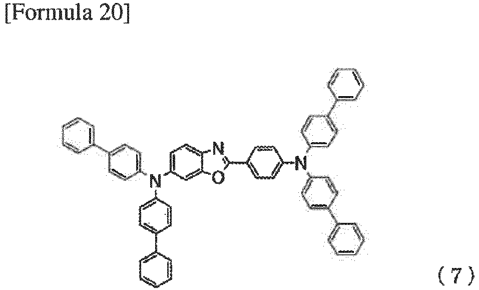

The structure of the yellow powder was identified using NMR.

The following 43 hydrogen signals were detected by ¹H-NMR(DMSO-d₆). δ(ppm)=8.06(2H),7.77-7.61(17H),7.51-7.41(9H),7.40-7.24(8H),7.21-7.12(7H).

### "Example 2"

### Synthesis of a compound <6-{(4-benzoxazole-2-yl-phenyl)-phenyl-amino}-2-[4-{(4-benzoxazole-2-yl-phenyl)-phenyl-amino}-phenyl]benzoxazole> represented by formula (22)

To a nitrogen-substituted reaction vessel was added 7.0 g of 6-bromo -2 (4-bromophenyl)-benzoxazole, 12.5 g of (4-benzoxazole-2-yl-phenyl)-phenyl-amine, 5.7 g of sodium t-butoxy, and 140 mL of toluene, and the vessel was aerated with nitrogen gas under ultrasonic irradiation for 30 minutes. To the reaction vessel was added 0.5 g of tris (dibenzylideneacetone) dipalladium (0) and 0.4 g of tri (tert-butyl) phosphine, and the mixture was stirred at reflux for 12 hours. The reaction vessel was allowed to cool, and then filtered and concentrated under reduced pressure to give a crude product.

The resulting crude product was purified by re-crystallization in a toluene/acetone mixed solvent to obtain 9.0 g (yield of 59%) of a yellow powder of 6-{(4-benzoxazole-2-yl-phenyl)-phenyl-amino}-2-[4-{(4-benzoxazole-2-yl-phenyl)-phenyl-amino}-phenyl]-benzoxazole (Compound represented by formula (22)).

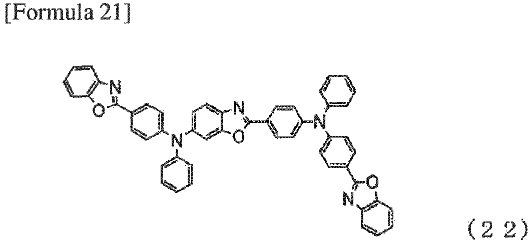

The structure of the yellow powder was identified using NMR.

The following 33 hydrogen signals were detected by ¹H-NMR(DMSO-d₆).

δ(ppm)=8.15(2H),8.10(2H),8.06(2H),7.81-7.69(5H),7.57(1H),7.51-7.36(8H),7.36-7.17(11 H),7.06(2H).

### "Example 3"

### Synthesis of a compound <5-(Biphenyl-4-yl-phenyl-amino)-2-{4-(Biphenyl-4-yl-phenyl-amino)-phenyl}benzoxazole> represented by formula (79)

To a nitrogen-substituted reaction vessel was added 7.0 g of 5-bromo -2 (4-bromophenyl)-benzoxazole, 10.7 g of biphenyl-4yl-phenyl-amine, 5.7 g of sodium t-butoxy, and 110 mL of toluene, and the vessel was aerated with nitrogen gas under ultrasonic irradiation for 30 minutes. To the reaction vessel was added 0.5 g of tris (dibenzylideneacetone) dipalladium (0) and 0.5 g of tri (tert-butyl) phosphine, and the mixture was stirred at reflux for 7 hours. The reaction vessel was allowed to cool, and then filtered and concentrated under reduced pressure to give a crude product.

The resulting crude product was purified by re-crystallization in a toluene/acetone mixed solvent to obtain 7.8 g (yield of 56%) of a yellow powder of 5-(biphenyl-4-yl-phenyl-amino)-2{4-(biphenyl-4-yl-phenyl-amino)-phenyl}-benzoxazole (Compound represented by formula (79)).

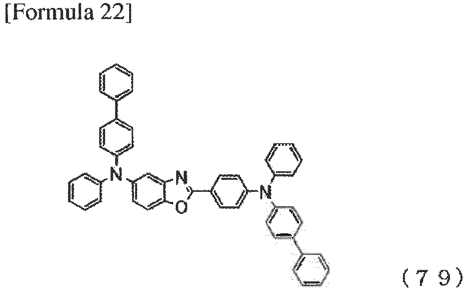

The structure of the yellow powder was identified using NMR.

The following 35 hydrogen signals were detected by ¹H-NMR(DMSO-d₆).

δ(ppm)=8.06(2H),7.71(3H),7.68(2H),7.62(4H),7.44(7H),7.34(4H),7.24(5H),7.08(8H).

### "Example 4"

### Synthesis of a compound <5-{(4-benzoxazole-2-yl-phenyl)-phenyl-amino}-2-[4-{(4-benzoxazole-2-yl-phenyl)-phenyl-amino]-phenyl]benzoxazole> represented by formula (93)

To a nitrogen-substituted reaction vessel was added 6.0 g of 5-bromo-2-(4-bromophenyl)-benzoxazole, 10.7 g of (4-benzoxazole-2-yl-phenyl)-phenyl-amine, 4.9 g of sodium t-butoxy, and 110 mL of toluene, and the vessel was aerated with nitrogen gas under ultrasonic irradiation for 30 minutes. To the reaction vessel was added 0.5 g of tris (dibenzylideneacetone) dipalladium (0) and 0.4 g of tri (tert-butyl) phosphine, and the mixture was stirred at reflux for 7 hours. The reaction vessel was allowed to cool, and then filtered and concentrated under reduced pressure to give a crude product.

The resulting crude product was purified by re-crystallization in a toluene solvent to obtain 6.0 g (yield of 46%) of a yellow powder of 5-{(4-benzoxazole-2-yl-phenyl)-phenyl-amino} -2[4-{ (4-benzoxazole-2-yl-phenyl)-phenyl-amino } -phenyl]-benzoxazole (Compound represented by formula (93)).

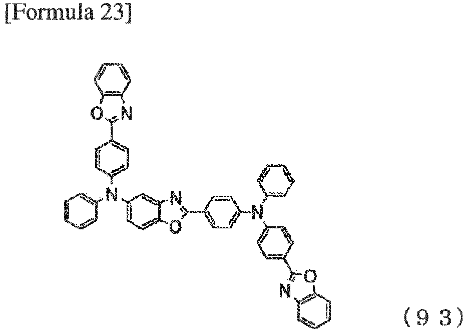

The structure of the yellow powder was identified using NMR.

The following 33 hydrogen signals were detected by ¹H-NMR(DMSO-d₆).

δ(ppm)=8.15(4H),8.05(2H),7.82(1H),7.78(2H),7.74(2H),7.61(1H),7.52-7.34(8H),7.34-7.17(11H),7.01(2H).

### "Example 5"

### Synthesis of a compound <4-(biphenyl-4-yl-phenyl-amino)-2-(4-(biphenyl-4-yl-phenyl-amino)-phenyl}benzoxazole> represented by formula (97)

To a nitrogen-substituted reaction vessel was added 7.0 g of 4-bromo-2-(4-bromophenyl)-benzoxazole, 10.7 g of biphenyl-4-yl-phenyl-amine, 5.7 g of sodium t-butoxy, and 110 mL of toluene, and the vessel was aerated with nitrogen gas under ultrasonic irradiation for 30 minutes. To the reaction vessel was added 0.5 g of tris (dibenzylideneacetone) dipalladium (0) and 0.5 g of tri (tert-butyl) phosphine, and the mixture was stirred at reflux for 4 hours. The reaction vessel was allowed to cool, and then filtered and concentrated under reduced pressure to give a crude product.

The resulting crude product was purified by column chromatography (Carrier: silica gel, eluent: n-heptane/toluene) to give 11.9 g (yield of 88%) of a yellow powder of 4-(biphenyl-4-yl-phenyl-amino)-2-(4-(biphenyl-4-yl-phenyl-amino)-phenyl)-benzoxazole (Compound represented by formula (97)).

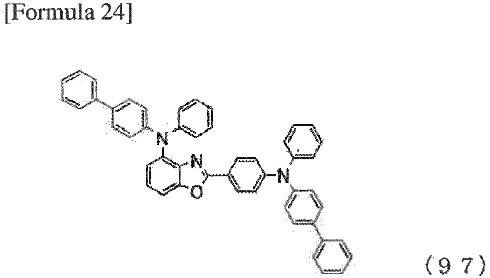

The structure of the yellow powder was identified using NMR.

The following 35 hydrogen signals were detected by ¹H-NMR (DMSO-d₆).

δ(ppm)=7.86(2H),7.69(2H),7.65(3H),7.61(2H),7.56(2H),7.51-7.26(11H),7.17(5H),7.06(8H).

### "Example 6"

### Synthesis of a compound <4-{(4-benzoxazole-2-yl-phenyl)-phenyl-amino}-2-[4-{(4-benzoxazole-2-yl-phenyl)-phenyl-amino}-phenyl]benzoxazole> represented by formula (99)

To a nitrogen-substituted reaction vessel was added 6.0 g of 4-bromo-2-(4-bromophenyl)-benzoxazole, 10.7 g of (4-benzoxazole-2-yl-phenyl)-phenyl-amine, 4.9 g of sodium t-butoxy, and 110 mL of toluene, and the vessel was aerated with nitrogen gas under ultrasonic irradiation for 30 minutes. To the reaction vessel was added 0.5 g of tris(dibenzylideneacetone) dipalladium (0) and 0.4 g of tri(tert-butyl) phosphine, and the mixture was stirred at reflux for 4 hours. The reaction vessel was allowed to cool, and then filtered and concentrated under reduced pressure to give a crude product.

The resulting crude product was purified by column chromatography (Carrier: silica gel, eluent: toluene/ethyl acetate) to give 7.1 g (yield of 55%) of a yellow powder of 4-{(4-benzoxazole-2-yl-phenyl)-phenyl-amino}-2[4-{(4-benzoxazole-2-yl-phenyl)-phenyl-amino}-phenyl]-benzoxazole (Compound represented by formula (99)).

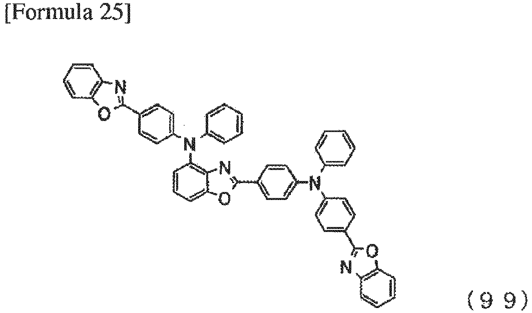

The structure of the yellow powder was identified using NMR.

The following 33 hydrogen signals were detected by ¹H-NMR (CDCl₃).

δ(ppm)=8.09(4H),7.92(2H),7.73(2H),7.54(2H),7.42-7.20(12H),7.21-7.07(11H).

### "Example 7"

### Synthesis of a compound <5-(biphenyl-4-yl-phenyl-amino)-2-{4-(biphenyl-4-yl-phenyl-amino)-phenyl}benzotriazole> represented by formula (101)

To a nitrogen-substituted reaction vessel was added 6.5 g of 5-bromo-2-(4-bromophenyl)-benzotriazole, 9.5 g of biphenyl -4-yl-phenyl-amine, 5.3 g of sodium t-butoxy, and 130 mL of toluene, and the vessel was aerated with nitrogen gas under ultrasonic irradiation for 30 minutes. To the reaction vessel was further added 0.1 g of palladium (II) acetate and 0.3 g of tri (tert-butyl) phosphine, and the mixture was stirred at reflux under heating for 7 hours. The reaction vessel was allowed to cool, and then filtered and concentrated under reduced pressure to give a crude product.

The resulting crude product was purified by re-crystallization in a toluene solvent to obtain 10.4 g (yield of 83%) of a yellow powder of 5-(biphenyl-4-yl-phenyl-amino)-2{4-(biphenyl-4-yl-phenyl-amino)-phenyl}-benzotriazole (Compound represented by formula (101)).

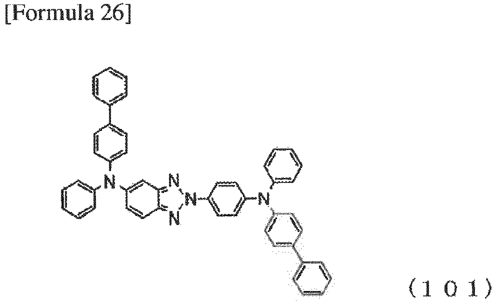

The structure of the yellow powder was identified using NMR.

The following 35 hydrogen signals were detected by ¹H-NMR(CDCl₃).

δ(ppm)=8.19(2H),7.82(1H),7.63(4H),7.57(4H),7.50(1H),7.47(4H),7.41-7.19(17H),7.15(2H).

### "Example 8"

### Synthesis of a compound <5-carbazole-9-yl-2-(4-Carbazole-9-yl-phenyl)-benzotriazole> represented by formula (102)

To a nitrogen-substituted reaction vessel was added 6.5 g of 5-bromo -2 (4-bromophenyl) -benzotriazole, 6.5 g of carbazole, 5.3 g of sodium t-butoxy, and 130 mL of toluene, and the vessel was aerated with nitrogen gas under ultrasonic irradiation for 30 minutes. To the reaction vessel was further added 0.1 g of palladium acetate and 0.3 g of tri (tert-butyl) phosphine, and the mixture was stirred under heated reflux overnight. The reaction vessel was allowed to cool, and then filtered and concentrated under reduced pressure to give a crude product.

The resulting crude product was purified by re-crystallization in a toluene solvent to obtain 4.7 g (yield of 37%) of a yellow powder of 5-carbazole-9-yl-2-(4-carbazole-9-yl-phenyl)-benzotriazole (Compound represented by formula (102)).

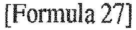

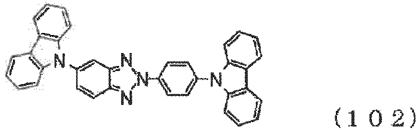

The structure of the yellow powder was identified using NMR.

The following 23 hydrogen signals were detected by ¹H-NMR (CDCl₃).

δ(ppm)=8.70(2H),8.22(6H),7.86(2H),7.71(1H),7.54(4H),7.50(4H),7.38(4H).

### (Measurement of Melting Point and Glass Transition Point (Tg) of Compounds)

The melting point and glass transition point (Tg) of each of the compounds represented by formula (7), (22), (79), (93), (97), (99), (101), and (102) synthesized in Examples 1 to 8 were measured using a highly sensitive differential scanning calorimeter (Bruker AXS, DSC3100SA). The results are shown below.

Example 1 (compound of formula (7)) Melting point; not observed, Tg; 138°C
Example 2 (compound of formula (22)) Melting point; not observed, Tg; 139°C
Example 3 (compound of formula (79)) Melting point; not observed, Tg; 104°C
Example 4 (compound of formula (93)) Melting point; not observed, Tg; 137°C
Example 5 (compound of formula (97)) Melting point; not observed, Tg; 106°C
Example 6 (compound of formula (79)) Melting point; not observed, Tg; 137°C
Example 7 (compound of formula (101)) Melting point; not observed, Tg; 105°C
Example 8 (compound of formula (102)) Melting point; 232°C, Tg; 115°C

The compounds represented by formula (7) (22) (79) (93) (97) (99) (101) (102) synthesized in Examples 1 to 8 have a glass transition point (Tg) of 100°C or higher, and are found to be materials capable of forming thin films with good stability.

### (Measurement of the Peak Wavelength, Absorbance, and Absorption Coefficient of A Compound)

The compounds of Formulas (7) (22) (79) (93) (97) (99) (101) (102) synthesized in Examples 1 to 8 were adjusted in toluene solvent to a concentration of 10⁻⁵ mol/L, respectively, and measured with an ultraviolet-visible near-infrared spectrophotometer (manufactured by JASCO Co., Ltd., V-650) in the range of 200 to 600 nm to determine the peak wavelength. The results are shown in Table 1.

The compounds of Formula (7) (22) (79) (93) (97) (99) (101) (102) synthesized in Examples 1 to 8 were adjusted to a concentration of 10⁻⁵ mol/L in a toluene solvent, respectively, and their absorbances were measured at 400 nm and 410 nm using an ultraviolet-visible near-infrared spectrophotometer (manufactured by JASCO Co., Ltd., V-650). The results are shown in Table 1.

Further, with respect to the compounds represented by formula (7) (22) (79) (93) (97) (99) (101) (102) synthesized in Examples 1 to 8, samples adjusted in toluene solution to four concentrations of 5.0×10⁻⁶ mol/L, 1.0×10⁻⁵ mol/L, 1.5×10⁻⁵ mol/L, and 2.0×10⁻⁵ mol/L were prepared, respectively. Absorbance at the peak wavelength of each sample was measured using an ultraviolet-visible near-infrared spectrophotometer (manufactured by JASCO Co., Ltd., V-650), and a calibration curve was prepared for each compound to calculate the absorption coefficient. The results are shown in Table 1.

For comparison, each peak wavelength, absorbance, and extinction coefficient were measured using a compound represented by formula (2-1) and Alq₃ in the same manner as the compound represented by formula (7).

The results are summarized in Table 1.

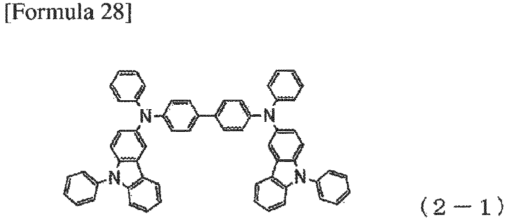

**[Table 1]**

| Table 1 | Peak wavelength (λ max) | Absorbance (λ:400 nm) | Absorbance (λ:400 nm) | Extinction coefficient |
|---|---|---|---|---|
| Compound (7) | 345nm | 1.43 | 1.25 | 153721 |
| Compound (22) | 396nm | 1.39 | 1.24 | 140500 |
| Compound (79) | 381nm | 0.76 | 0.38 | 105406 |
| Compound (93) | 383nm | 1.32 | 0.77 | 149101 |
| Compound (97) | 323nm | 0.73 | 0.42 | 114838 |
| Compound (99) | 372nm | 1.10 | 0.80 | 131494 |
| Compound (101) | 324nm | 1.21 | 1.36 | 146510 |
| Compound (102) | 243nm | 0.27 | 0.07 | 196092 |
| Comparative compound (2-1) | 358nm | 0.07 | 0.02 | 48856 |
| Alq₃ | 394nm | 0.07 | 0.06 | 7518 |

As shown in Table 1, the peak wavelength of the compound represented by formula (7) (22) (79) (93) (97) (99) (101) (102) was 400 nm or less, which is the same as those of the compound represented by formula (2-1) and Alq₃. This indicates that for the compound represented by formula (7) (22) (79) (22) (79) (93) (97) (99), it is difficult to absorb light in the respective wavelength regions of blue, green, and red, and can be used as a material for a thin film which absorbs light at wavelengths of 400 nm and 410 nm.

As shown in Table 1, the compound represented by formula (7) (22) (79) (93) (97) (99) (101) (102) had a higher absorbance than the compound represented by formula (2-1) and Alq₃. From this, it was found that the compound represented by formula (7) (22) (79) (93) (97) (99) (101) (102) is a material capable of forming a thin film having a good function of absorbing light at wavelengths of 400 nm and 410 nm as compared with the compound represented by formula (101) and Alq₃ at the same concentration,

The compound represented by formula (7) (22) (79) (93) (97) (99) (101) (102) had a larger absorption coefficient than the compound represented by formula (2-1) and Alq₃. Therefore, by using a compound represented by formula (7) (22) (79) (22) (79) (93) (97) (99) (101) (102), it is possible to form a film in which the function of absorbing light remarkably increases as the film thickness is increased.

### (Measurement of Refractive Index and Extinction Coefficient of Thin Film)

A vapor-deposited film having a thickness of 80 nm was formed on a silicon substrate by using each of the compounds represented by formula (7) (22) (79) (93) (97) (99) (101) (102) synthesized in Examples 1 to 8.

With respect to the thus obtained vapor-deposited films, light having a wavelength of 400 nm and light having a wavelength of 410 nm were transmitted to the capping layer and reflected on the silicon substrate, respectively, by measurement using a spectrophotometer (Filmetrics, F 10 RT-UV), and the refractive index n and the extinction coefficient k of the capping layer were obtained. The results are shown in Table 2.

For comparison, a vapor-deposited film having a thickness of 80 nm was formed on a silicon substrate by using a compound represented by formula (2-1) and Alq₃, and light having a thickness of 400 nm and light having a thickness of 410 nm were transmitted to a capping layer and reflected on the silicon substrate, respectively, in the same manner as the vapor-deposited film of the compound represented by formula (7), and the refractive index n and the extinction coefficient k of the capping layer were obtained (for example, see Patent Document 4). The results are summarized in Table 2.

**[Table 2]**

| Table 2 | Refractive index n (λ 400 nm) | Refractive index n (λ 410 nm) | Extinction coefficient k (λ 400 nm) | Extinction coefficient k (λ 410 nm) |
|---|---|---|---|---|
| Compound (7) | 2.13 | 2.20 | 0.52 | 0.44 |
| Compound (22) | 2.15 | 2.34 | 0.85 | 0.73 |
| Compound (79) | 2.24 | 2.33 | 0.53 | 0.37 |
| Compound (93) | 2.41 | 2.58 | 0.90 | 0.67 |
| Compound (97) | 2.23 | 2.30 | 0.49 | 0.33 |
| Compound (99) | 2.29 | 2.39 | 0.69 | 0.52 |
| Compound (101) | 2.61 | 2.68 | 0.81 | 0.48 |
| Compound (102) | 2.25 | 2.31 | 0.35 | 0.21 |
| Comparative compound (2-1) | 2.13 | 2.10 | 0.15 | 0.06 |
| Alq₃ | 1.86 | 1.89 | 0.16 | 0.14 |

As shown in Table 2, the thin film made of the compound represented by formula (7) (22) (79) (93) (97) (99) (101) (102) had a refractive index n equal to or higher than that of the thin film made of the compound represented by formula (2-1) and was higher than that of the thin film made of Alq₃.

Therefore, by using a thin film made of a compound represented by formula (7) (22) (79) (93) (97) (99) (101) (102) as a capping layer, it can be expected to improve the light extraction efficiency in the organic EL element.

The thin film made of the compound represented by formula (7) (22) (79) (93) (97) (99) (101) (102) had a higher extinction coefficient k than the thin film made of the compound represented by formula (2-1) and Alq₃.

This shows that the capping layer using a thin film of a compound represented by formula (7) (22) (79) (93) (97) (99) (101) (102) absorbs light having a wavelength of 400 nm to 410 nm well, and does not affect the material inside the element.

### "Example 9"

The organic EL element shown in FIG. 1 was manufactured by the following method.

A hole injection layer (3), a hole transport layer (4), a light emitting layer (5), an electron transport layer (6), an electron injection layer (7), a cathode (8), and a capping layer (9) were formed in this order by a vapor deposition method on a glass substrate (1) on which a reflection electrode was previously formed as an anode (2) made of metal, thereby obtaining an organic EL element shown in FIG. 1.

Specifically, a glass substrate 1 was prepared in which ITO with a film thickness of 50 nm, a reflective film of a silver alloy with a film thickness of 100 nm, and ITO with a film thickness of 5 nm were sequentially formed as anodes 2. The glass substrate 1 was subjected to ultrasonic cleaning in isopropyl alcohol for 20 minutes and then dried on a hot plate heated to 250°C for 10 minutes. Then, after performing UV ozone treatment for 2 minutes, the glass substrate 1 with ITO was attached to a vacuum vapor deposition apparatus, and the vacuum vapor deposition apparatus was depressurized to 0.001 Pa or less.

Subsequently, as the hole injection layer 3 covering the anode 2, an electron acceptor (Acceptor-1) and a compound (3-1) represented by the following formula were subjected to binary vapor deposition at a vapor deposition rate such that the ratio of the vapor deposition rate was Acceptor-1: compound (3-1) = 3:97 to form a film thickness of 10 nm.

On the hole injection layer 3, a compound (3-1) was formed as the hole transport layer 4 so as to have a film thickness of 140 nm.

On the hole transport layer 4, a compound represented by the following formula (3-2) and a compound represented by the following formula (3-3) were binary vapor-deposited as the light emitting layer 5 at a deposition rate such that the deposition rate ratio of the compound (3-2): compound (3-3) was 5: 95 to form a film thickness of 20 nm.

On the light emitting layer 5, a compound represented by the following formula (3-4) and a compound represented by the following formula (3-5) were deposited as the electron transport layer 6 at a deposition rate such that the deposition rate ratio of the compound (3-4): compound (3-5) was 50: 50 to form a film thickness of 30 nm.

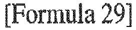

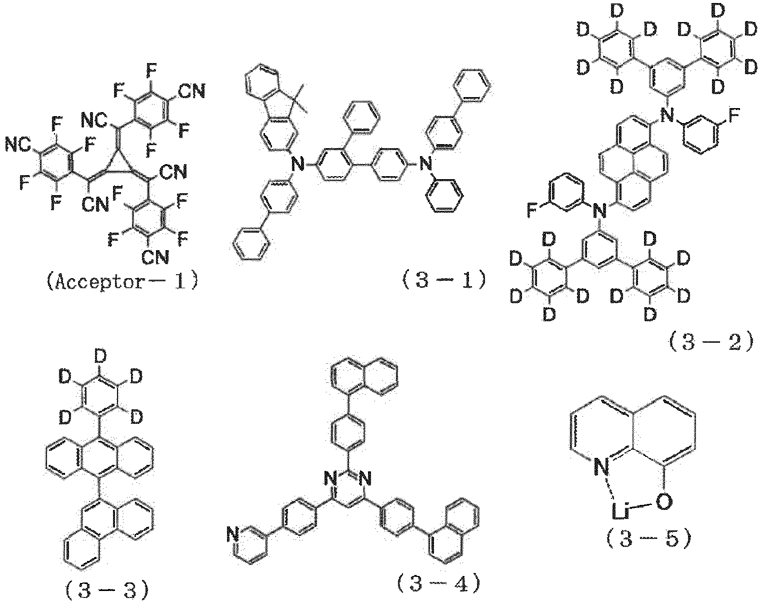

On the electron transport layer 6, lithium fluoride was formed as an electron injection layer 7 with a thickness of 1 nm.

On the electron injection layer 7, a magnesium-silver alloy was formed to have a film thickness of 12 nm as a translucent cathode 8.

Finally, the compound represented by formula (7) synthesized in Example 1 was deposited on the cathode 8 to form a capping layer 9 having a film thickness of 60 nm, thereby obtaining the organic EL element of Example 9.

### "Example 10"

An organic EL element of Example 10 was obtained in the same manner as in Example 9 except that a compound of Formula (22) synthesized in Example 2 was used as a material for the capping layer 9 in place of the compound of Formula (7) synthesized in Example 1.

### "Example 11"

An organic EL element of Example 11 was obtained in the same manner as in Example 9 except that a compound of Formula (79) synthesized in Example 3 was used as a material for the capping layer 9 in place of the compound of Formula (7) synthesized in Example 1.

### "Example 12"

An organic EL element of Example 12 was obtained in the same manner as in Example 9 except that a compound of Formula (93) synthesized in Example 4 was used as a material for the capping layer 9 in place of the compound of Formula (7) synthesized in Example 1.

### "Example 13"

An organic EL element of Example 13 was obtained in the same manner as in Example 9 except that a compound of Formula (97) synthesized in Example 5 was used as a material for the capping layer 9 in place of the compound of Formula (7) synthesized in Example 1.

### "Example 14"

An organic EL element of Example 14 was obtained in the same manner as in Example 9 except that a compound of Formula (99) synthesized in Example 6 was used as a material for the capping layer 9 in place of the compound of Formula (7) synthesized in Example 1.

### "Example 15"

An organic EL element of Example 15 was obtained in the same manner as in Example 9 except that a compound of Formula (101) synthesized in Example 7 was used as a material for the capping layer 9 in place of the compound of Formula (7) synthesized in Example 1.

### "Example 16"

An organic EL element of Example 16 was obtained in the same manner as in Example 9 except that a compound of Formula (102) synthesized in Example 8 was used as a material for the capping layer 9 in place of the compound of Formula (7) synthesized in Example 1.

### "Comparative Example 1"

An organic EL element of Comparative Example 1 was obtained in the same manner as in Example 9 except that a compound of the above formula (2 -1) was used as a material of the capping layer 9 in place of the compound of the formula (7) synthesized in Example 1.

### "Comparative Example 2"

An organic EL element of Comparative Example 2 was obtained in the same manner as in Example 9 except that Alq₃ was used as the material of the capping layer 9 in place of the compound of Formula (7) synthesized in Example 1.

The characteristics of the organic EL elements of Examples 9 to 16, Comparative Example 1, and Comparative Example 2 were measured in the air and at room temperature, respectively.

Specifically, the driving voltage, luminance, light emission efficiency, and power efficiency at a current density of 10 mA/cm² were measured when a DC voltage was applied to each organic EL element. The results are shown in Table 3.

The organic EL elements of Examples 9 to 16, Comparative Example 1, and Comparative Example 2 were driven at a constant current of 10 mA/cm² at room temperature in the atmosphere, respectively, and the time until the luminance attenuated to 95% when the initial luminance was 100% was measured, and evaluated as the life (element lifetime). The results are shown in Table 3.

**[Table 3]**

| Table 3 | Capping layer | Voltage [V] (10mA/ cm²) | Luminance [cd/m²] (10mA/ cm²) | Light Power emission efficiency Element efficiency [lm/W] life [cd/A] 10mA/ 95% (10mA/ cm²) decay cm²) | | |
|---|---|---|---|---|---|---|
| Example 9 | Compound (7) | 3.65 | 714 | 7.14 | 6.15 | 156 hours |
| Example 10 | Compound (22) | 3.64 | 719 | 7.19 | 6.21 | 181 hours |
| Example 11 | Compound (79) | 3.63 | 736 | 7.36 | 6.37 | 171 hours |
| Example 12 | Compound (93) | 3.63 | 742 | 7.43 | 6.43 | 174 hours |
| Example 13 | Compound (97) | 3.65 | 733 | 7.33 | 6.31 | 193 hours |
| Example 14 | Compound (99) | 3.67 | 738 | 7.38 | 6.32 | 188 hours |
| Example 15 | Compound (101) | 3.68 | 779 | 7.78 | 6.65 | 169 hours |
| Example 16 | Compound (102) | 3.63 | 733 | 7.33 | 6.34 | 184 hours |
| Comparative Example 1 | Comparative compound (2-1) | 3.69 | 668 | 6.68 | 5.68 | 121 hours |
| Comparative Example 2 | Alq₃ | 3.67 | 647 | 6.47 | 5.54 | 106 hours |

As shown in Table 3, the driving voltage at a current density of 10 mA/cm² was almost the same in the organic EL elements of Examples 9 to 16, Comparative Example 1, and

### Comparative Example 2.

As shown in Table 3, the organic EL elements of Examples 9 to 16 had higher luminance, light emission efficiency, and power efficiency than those of Comparative Examples 1 and 2. This shows that by using a compound represented by fonnula (7) (22) (79) (93) (97) (99) (101) (102) as a material for the capping layer of the organic EL element, the light extraction efficiency can be greatly improved as compared with the case where the compound represented by formula (2-1) and Alq₃ are used.

The organic EL elements of Examples 9 to 16 had a longer life than those of Comparative Examples 1 and 2.

In particular, the organic EL element of Example 13 using the compound of Formula (97) as the material of the capping layer had a long life.

### INDUSTRIAL APPLICABILITY

As described above, in the organic EL element of the present invention, the diamine compound represented by general formula (a-1) having a benzazole ring structure has a high absorption coefficient, a high refractive index, and a stable thin film state. Therefore, it is excellent as a compound used for the capping layer of the organic EL element. The organic EL element having a capping layer using the compound can obtain high luminous efficiency, light extraction efficiency, and power efficiency. Further, by forming the organic EL element having the capping layer using the compound, durability and light resistance can be improved so as not to absorb the light of sunlight and affect the material inside the element.

The diamine compound represented by general formula (a-1) having a benzazole ring structure has no absorption in each wavelength region of blue, green, and red. Therefore, the organic EL element having the capping layer using the compound is particularly suitable for displaying a clear and bright image having a good color purity. For example, it has become possible to apply it to home appliances and lighting applications.

### [Description of the Signs]

1. Glass Substrate
2. Anode
3. Hole Injection Layer
4. Hole Transport Layer
5. Light emitting Layer
6. Electron Transport Layer
7 Electron Injection Layer
8. Cathode
9. Capping Layer

## Claims

1. An organic electroluminescent element comprising an organic layer comprising a light emitting layer which is provided between a first electrode and a second electrode,
wherein a capping layer is laminated on a surface of the first electrode opposite to the organic layer;
the first electrode is transparent or translucent; and
the capping layer comprises a diamine compound having a benzazole ring structure represented by general formula (a-1), 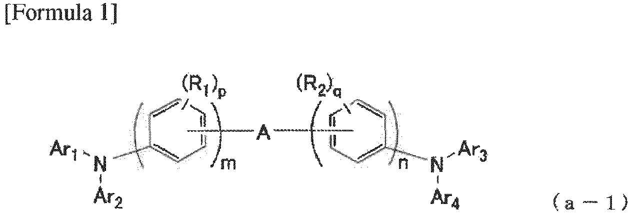 wherein in formula (a-1),
A represents a divalent group represented by the following general formula (b-1) wherein the divalent group has two binding sites among R₃ to R₁₂;
Ar₁ to Ar₄ may be the same or different from each other, and each represents a monovalent group represented by general formula (b-1) wherein the monovalent group has one binding site at R₃ to R₁₂, a substituted or unsubstituted aromatic hydrocarbon group, a substituted or unsubstituted aromatic heterocyclic group, or a substituted or unsubstituted condensed polycyclic aromatic group;
R₁ and R₂ may be the same or different from each other, and may represent hydrogen, deuterium, fluorine, chlorine, cyano, nitro, trimethylsilyl, triphenylsilyl, a linear or branched alkyl having 1 to 6 carbon atoms optionally substituted, a cycloalkyl having 5 to 10 carbon atoms optionally substituted, a linear or branched alkenyl having 2 to 6 carbon atoms opionally substituted, a linear or branched alkyloxy having 1 to 6 carbon atoms optionally substituted, a cycloalkyloxy having 5 to 10 carbon atoms optionally substituted, a substituted or unsubstituted aryloxy, a substituted or unsubstituted aromatic hydrocarbon, a substituted or unsubstituted aromatic heterocyclic, or a substituted or unsubstituted condensed polycyclic aromatic group;
m and n represent integers of 0 to 2, and p and q represent integers of 0 to 4;
when m or n is 0, A is bonded to a nitrogen atom as a single bond;
when m and n are integers of 1 or more, Ar₁, Ar₂, and the benzene ring adjacent to their bonded nitrogen atoms, or Ar₃, Ar₄, and the benzene ring adjacent to their bonded nitrogen atoms may be bonded via the nitrogen atom by a single bond, a substituted or unsubstituted methylene group, a substituted or unsubstituted amino group, an oxygen atom, or a sulfur atom to form a ring;
when p and q are integers of 2 or more, plurality of R₁ and R₂ bonded to the same benzene ring may be identical or different from each other, and may be bonded to each other through a single bond, a substituted or unsubstituted methylene group, a substituted or unsubstituted amino group, an oxygen atom, or a sulfur atom to form a ring with respect to the same substituted benzene ring, 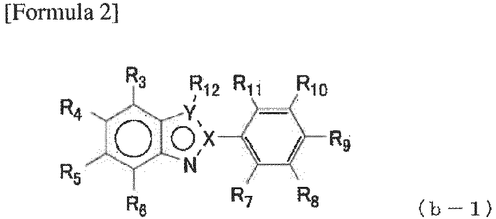 wherein in formula (b-1),
R₃ to R₁₂ may be the same or different from each other, and each may represent a linking group as a binding site, a hydrogen atom, a deuterium atom, a fluorine atom, a chlorine atom, a cyano group, a nitro group, a trimethylsilyl group, a triphenylsilyl group, a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group;
X represents a carbon atom or a nitrogen atom;
Y represents any one of carbon, nitrogen, oxygen, and sulfur atoms;
provided that, when X is a carbon atom and Y is an oxygen atom, when X is a carbon atom and Y is a sulfur atom, or when X is a nitrogen atom and Y is a nitrogen atom, Y does not have R₁₂.

2. The organic electroluminescent element according to claim 1,
wherein the first electrode is a cathode;
the second electrode is an anode;
the organic layer includes at least a hole transport layer, a light emitting layer, and an electron transport layer; and
the organic EL element comprises the anode, the hole transport layer, the light emitting layer, the electron transport layer, the cathode, and the capping layer, in this order.

3. The organic electroluminescent element according to claim 1, wherein the general formula (b-1) is the following general formula (b-2), (b-3), or (b-4), 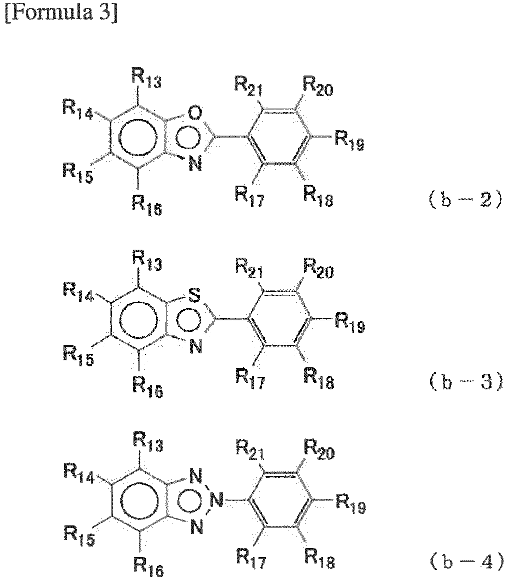 wherein in fonnulas (b-2), (b-3), or (b-4), R₁₃ to R₂₁ may be the same or different from each other, and each may represent a linking group as a binding site, a hydrogen atom, a deuterium atom, a fluorine atom, a chlorine atom, a cyano group, a nitro group, a trimethylsilyl group, a triphenylsilyl group, a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group.

4. The organic electroluminescent element according to claim 1, wherein the general formula (b-1) is the following general formula (b-5), (b-6), or (b-7), 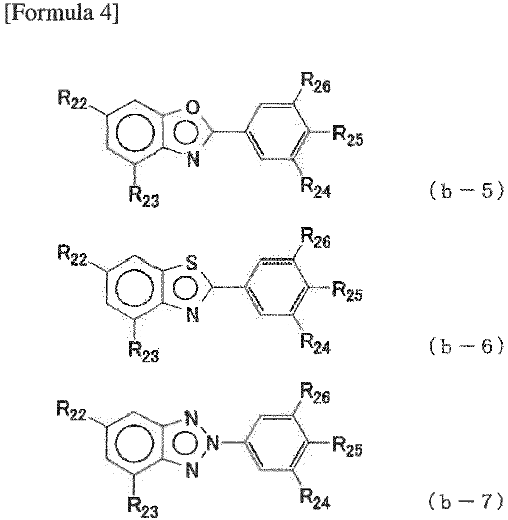 wherein in formulas (b-5),(b-6), or (b-7), R₂₂ to R₂₆ may be the same or different from each other, and each may represent a linking group as a binding site, a hydrogen atom, a deuterium atom, a fluorine atom, a chlorine atom, a cyano group, a nitro group, a trimethylsilyl group, a triphenylsilyl group, a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group.

5. The organic electroluminescent element according to claim 1, wherein the general formula (b-1) is the following general formula (b-8), (b-9), or (b-10), 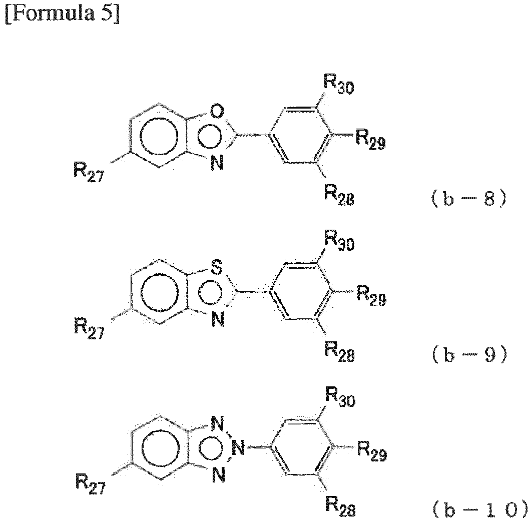 wherein in formulas (b-8), (b-9), or (b-10), R₂₇ to R₃₀ may be the same or different from each other, and each may represent a linking group as a binding site, a hydrogen atom, a deuterium atom, a fluorine atom, a chlorine atom, a cyano group, a nitro group, a trimethylsilyl group, a triphenylsilyl group, a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group.

6. The organic electroluminescent element according to any one of claims 1 to 5, wherein the sum of m and n is 0, 1, or 2.

7. The organic electroluminescent element according to any one of claims 1 to 6, wherein the two amino group skeletons of -NAr₁Ar₂ and NAr₃Ar₄ in the general formula (a-1) are the same.

8. The organic electroluminescent element according to any one of claims 1 to 7, wherein the diamine compound has an absorbance of 0.2 or more in the absorption spectrum of concentration of 10⁻⁵ mol/L in a wavelength range of 400 nm to 410 nm.

9. The organic electroluminescent element according to any one of claims 1 to 8, wherein the thickness of the capping layer is in the range of 30 nm to 120 nm.

10. The organic electroluminescent element according to any one of claims 1 to 9, wherein the capping layer has a refractive index of 1.85 or more when light having wavelengths of 400 nm and 410 nm is transmitted.

11. The organic electroluminescent element according to any one of claims 1 to 10, wherein the capping layer has an extinction coefficient of 0.2 or more for light having wavelengths of 400 nm and 410 nm.

12. The organic electroluminescent element according to any one of claims 1 to 11, wherein R1 and R₂ are hydrogen atoms.
